Europäisches Patentamt

European Patent Office

Office européen des brevets

(19)

(11) Publication number: **0 382 990**

**A1**

(12)

# EUROPEAN PATENT APPLICATION

(21) Application number: **89400428.2**

(51) Int. Cl.5: **C12N 15/32, A01N 63/00**

(22) Date of filing: **15.02.89**

(43) Date of publication of application:
**22.08.90 Bulletin 90/34**

(84) Designated Contracting States:
**AT BE CH DE ES FR GB GR IT LI LU NL SE**

(71) Applicant: **PLANT GENETIC SYSTEMS, N.V.**
**Kolonel Bourgstraat 106 Bus 1**
**B-1040 Brussel(BE)**

(72) Inventor: **Peferoen, Marnix**
**J.P. Minckelersstraat 129**
**B-3000 Leuven(BE)**
Inventor: **Lambert, Bart**
**Zuidveldstraat 1**
**B-8030 Beernem(BE)**
Inventor: **Joos, Henk**
**Oostmolen Zuid 5**
**B-9880 Aalter(BE)**

(74) Representative: **Gutmann, Ernest et al**
**S.C. Ernest Gutmann - Yves Plasseraud 67,**
**boulevard Haussmann**
**F-75008 Paris(FR)**

(54) Strains of bacillus thuringiensis.

(57) Two new Bacillus thuringiensis strains, which are deposited at the DSM under accession nos. 5131 and 5132, produce crystal proteins during sporulation that are toxic to Coleoptera. The crystal proteins contain 74 kDa and 129 kDa protoxins, respectively, which can yield 67 and 66 kDa toxins, respectively, as trypsin-digestion products. A plant, the genome of which is transformed with a DNA sequence that comes from either one of the strains and that codes for its respective toxin, is resistant to Coleoptera. Each strain, itself, or its crystals, crystal proteins, protoxin or toxin can be used as the active ingredient in an insecticidal composition for combatting Coleoptera.

EP 0 382 990 A1

## NEW STRAINS OF BACILLUS THURINGIENSIS

This invention relates to two new strains of B. thuringiensis (the "BtPGSI208 strain" and the "BtPGSI245 strain"), each of which produces crystallized proteins (the "BtPGSI208 crystal proteins" and the "BtPGSI245 crystal proteins", respectively) which are packaged in crystals (the "BtPGSI208 crystals" and the "BtPGSI245 crystals", respectively) during sporulation. The BtPGSI208 and BtPGSI245 strains were deposited under the provisions of the Budapest Treaty at the Deutsche Sammlung Für Mikroorganismen and Zellkulturen ("DSM"), Mascheroder Weg 1B, D-3300 Braunschweig, Federal Republic of Germany, under accession numbers 5131 and 5132, respectively, on January 19, 1989.

This invention also relates to an insecticide composition that is active against Coleoptera and that comprises the BtPGSI208 or BtPGSI245 strain, as such, or preferably the BtPGSI208 or BtPGSI245 crystals, crystal proteins or the active component(s) thereof as an active ingredient.

This invention further relates to:

1) a DNA sequence (the "btPGSI208 gene"), from the genome of the BtPGSI208 strain, which encodes a 74 kDa protein (the "BtPGSI208 protoxin") that is found in the BtPGSI208 crystals; and

2) A DNA sequence (the "btPGSI245 gene), from the genome of the BtPGSI245 strain, which encodes a 129 kDa protein (the "BtPGSI245 protoxin") that is found in the BtPGSI245 crystals. The BtPGSI208 and BtPGSI245 protoxins are the proteins that are produced by their respective BtPGSI208 and BtPGSI245 strains before being packaged into their respective BtPGSI208 and BtPGSI245 crystals.

This invention still further relates to a 67 kDa protein ("the BtPGSI208 toxin") and a 66 kDa protein (the "BtPGSI245 toxin") which can be obtained from the BtPGSI208 protoxin and the BtPGSI245 protoxin, respectively. The BtPGSI208 and BtPGSI245 toxins are insecticidally active proteins which can be liberated from the BtPGSI208 crystals and the BtPGSI245 crystals, respectively, produced by the BtPGSI208 strain and the BtPGSI245 strain, respectively, and each toxin has a high activity against Coleoptera. The BtPGSI208 and BtPGSI245 toxins are believed to represent the smallest portions of their respective BtPGSI208 and BtPGSI245 protoxins which are insecticidally effective against Coleoptera.

This invention yet further relates to a chimaeric gene that can be used to transform a plant cell and that contains:

1) a part of the btPGSI208 or btPGSI245 gene (the "insecticidally effective btPGSI208 or btPGSI245 gene part") encoding an insectidicidally effective portion of the respective BtPGSI208 or BtPGSI245 protoxin, preferably a truncated part of the btPGSI208 or btPGSI245 gene (the "truncated btPGSI208 or btPGSI245 gene") encoding just the respective BtPGSI208 or BtPGSI245 toxin;

2) a promoter suitable for transcription of the insecticidally effective btPGSI208 or btPGSI245 gene part in a plant cell; and

3) suitable transcription termination and polyadenylation signals for expressing the insecticidally effective btPGSI208 or btPGSI245 gene part in a plant cell. This chimaeric gene is hereinafter generally referred to as the "btPGSI208 or btPGSI245 chimaeric gene." Preferably, the insecticidally effective btPGSI208 or btPGSI245 gene part is present in the btPGSI208 or btPGSI245 chimaeric gene as a hybrid gene comprising a fusion of the truncated btPGSI208 or btPGSI245 gene and a selectable marker gene, such as the neo gene (the "btPGSI208-neo or btPGSI245-neo hybrid gene").

This invention also relates to:

1) a cell (the "transformed plant cell") of a plant, such as potato, the genome of which is transformed with the insecticidally effective btPGSI208 or btPGSI245 gene part; and

2) a plant (the "transformed plant") which is regenerated from the transformed plant cell or is produced from the so-regenerated plant, the genome of which contains the insecticidally effective btPGSI208 or btPGSI245 gene part and which is resistant to Coleoptera.

## Background of the Invention

B. thuringiensis ("Bt") is a gram-positive bacterium which produces endogenous crystals upon sporulation. The crystals are composed of proteins which are specifically toxic against insect larvae. Three different Bt pathotypes have been described: pathotype A that is active against Lepidoptera, e.g., caterpillars; pathotype B that is active against certain Diptera, e.g., mosquitos and black flies; and pathotype C that is active against Coleoptera, e.g., beetles (Ellar et al, 1986).

A Bt strain, whose crystals are toxic to Coleoptera, has been described as Bt tenebrionis (US patent 4,766,203; European patent publication 0,149,162), Bt M-7 or Bt San Diego (European patent publication 0,213,818; U.S. patent 4,771,131) and BtS1 (European patent application 88/402,115.5).

The fact that conventional submerged fermentation techniques can be used to produce Bt spores on a large scale makes Bt bacteria commercially attractive as a source of insecticidal compositions.

Gene fragments from some Bt strains, encoding insecticidal proteins, have heretofore been identified and integrated into plant genomes in order to render the plants insect-resistant. However, obtaining expression of such Bt gene fragments in plants is not a straightforward problem. To achieve optimal expression of an insecticidal protein in plant cells, it has been found necessary to engineer each Bt gene fragment in a specific way so that it encodes a water-soluble part of a Bt protoxin that retains substantial toxicity against its target insects (European patent applications 86/300,291.1 and 88/402,115.5; U.S. patent application 821,582, filed January 22, 1986)

Summary of the Invention

In accordance with this invention, the two new Bt strains of pathotype C, i.e., the BtPGSI208 and BtPGSI245 strains, are provided. The BtPGSI208 and BtPGSI245 crystals, crystal proteins, protoxins and toxins, produced by the respective strains during sporulation, as well as insecticidally effective portions of the BtPGSI208 and BtPGSI245 protoxins, each possess insecticidal activity and can therefore be formulated into insecticidal compositions against Coleoptera in generally especially against Leptinotarsa decemlineata, Agelastica alni, Diabrotica luteola, Haltica tombacina, Anthonomus grandis, Tenebrio molitor and Triboleum castaneum and particularly against the Colorado potato beetle, Leptinotarsa decemlineata, which is a major pest of economically important crops.

Also in accordance with this invention, a plant cell genome is transformed with the insecticidally effective btPGSI208 or btPGSI245 gene part, preferably the truncated btPGSI208 or btPGSI245 gene. It is preferred that this transformation be carried with the btPGSI208 or btPGSI245 chimaeric gene. The resulting transformed plant cell can be used to produce a transformed plant in which the plant cells in some or all of the plant tissues: 1) contain the insecticidally effective btPGSI208 or btPGSI245 gene part as a stable insert in their genome and 2) express the insecticidally effective btPGSI208 or btPGSI245 gene part by producing an insecticidally effective portion of its respective BtPGSI208 or BtPGSI245 protoxin, preferably its respective BtPGSI208 or BtPGSI245 toxin, thereby rendering the plant resistant to Coleoptera.

Yet further in accordance with this invention, a process is provided for rendering a plant resistant to Coleoptera by transforming the plant cell genome with the insecticidally effective btPGSI208 or btPGSI245 gene part, preferably the truncated btPGSI208 or btPGSI245 gene. In this regard, it is preferred that the plant cell be transformed with the btPGSI208 or btPGSI245 chimaeric gene.

Still further in accordance with this invention, there are provided the BtPGSI208 and BtPGSI245 protoxins, the insecticidally effective portions of such protoxins and the BtPGSI208 and BtPGSI245 toxins, as well as the btPGSI208 and btPGSI245 genes, the insecticidally effective btPGSI208 and btPGSI245 gene parts, the truncated btPGSI208 and btPGSI245 genes and the chimaeric btPGSI208 and btPGSI245 genes.

Detailed Description of the Invention

In accordance with this invention, the BtPGSI208 and BtPGSI245 protoxins can be isolated in a conventional manner from, respectively, the BtPGSI208 strain, deposited at the DSM under accession number 5131, and the BtPGSI245 strain, deposited at the DSM under accession number 5132. For example, the BtPGSI208 and BtPGSI245 crystals can be isolated from sporulated cultures of their respective strains (Mahillon and Delcour, 1984), and then, the respective protoxins can be isolated from these crystals according to the method of Höfte et al (1986). The protoxins can be used to prepare monoclonal or polyclonal antibodies specific for these protoxins in a conventional manner (Höfte et al, 1988). The BtPGSI208 toxin can then be obtained by removing (e.g., by trypsin digestion) approximately 57 N-terminal amino acids from the BtPGSI208 protoxin. The BtPGSI245 toxin can be obtained by removing (e.g., by trypsin digestion) approximately 52 N-terminal and approximately 501 C-terminal amino acids from the BtPGSI245 protoxin.

The btPGSI208 and btPGSI245 genes can also be isolated from their respective strains in a conventional manner. For example, the btPGSI208 or btPGSI245 gene can be identified in its respective BtPGSI208 or BtPGSI245 strain, using the procedure described in U.S. patent application 821,582 and in European patent applications 86/300,291.1 and 88/402,115.5 (which are incorporated herein by reference). Preferably, the btPGSI208 and btPGSI245 genes are each identified by: digesting total DNA from their

3

respective BtPGSI208 and BtPGSI245 strains with one or more restriction enzymes; size fractionating the DNA fragments, so produced, into DNA fractions of 5 to 10 Kb; ligating such fractions to cloning vectors; transforming E. coli with the cloning vectors; and screening the clones with a suitable DNA probe. The DNA probe can be constructed: 1) from a highly conserved region of a Bt gene which codes for another crystal protoxin against Coleoptera such as: the bt13 gene described in European patent application 88/402,115.5 and by Höfte et al (1987); or 2) on the basis of the N-terminal amino acid sequence of the protoxin encoded by the respective btPGSI208 or btPGSI245 gene, which sequence can be determined by gas-phase sequencing of the immobilized protoxin (European Patent application 88/402,115.5).

Alternatively, the 5 to 10 kB fragments, prepared from total DNA of the BtPGSI208 or BtPGSI245 strain, can be ligated in suitable expression vectors and transformed in E. coli, and the clones can then be screened by conventional colony immunoprobing methods (French et al, 1986) for expression of the BtPGSI208 or BtPGSI245 toxin with monoclonal or polyclonal antibodies raised against the toxin.

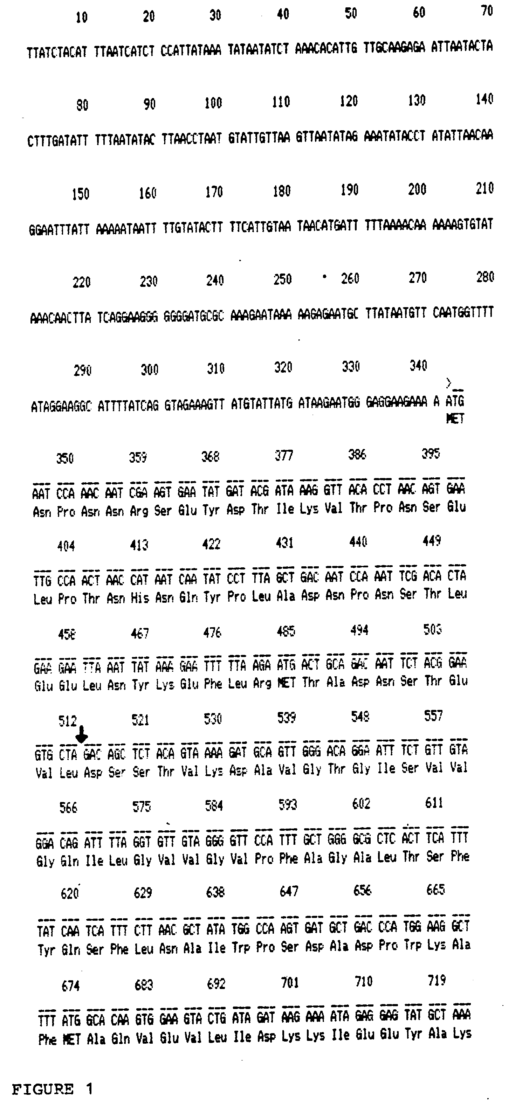

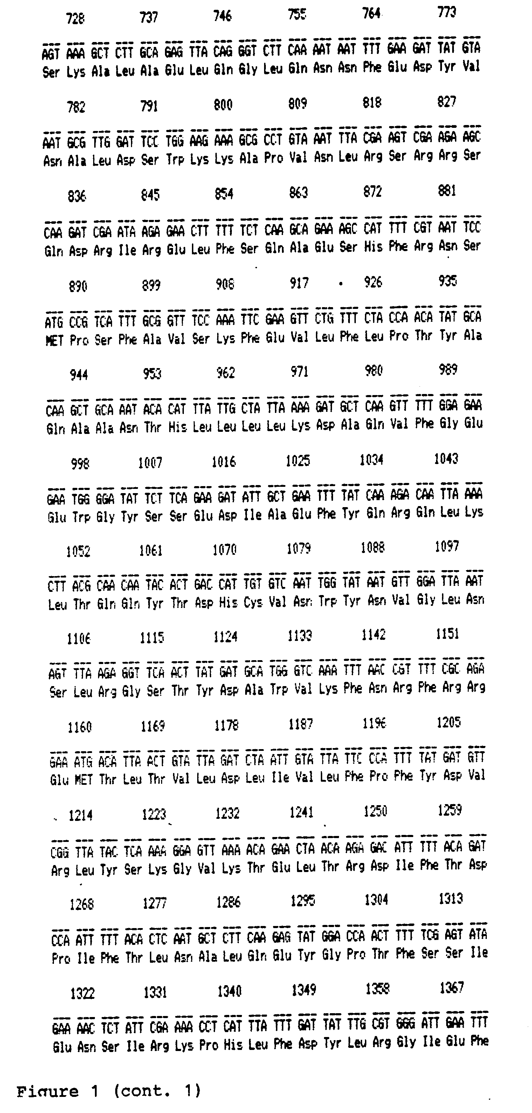

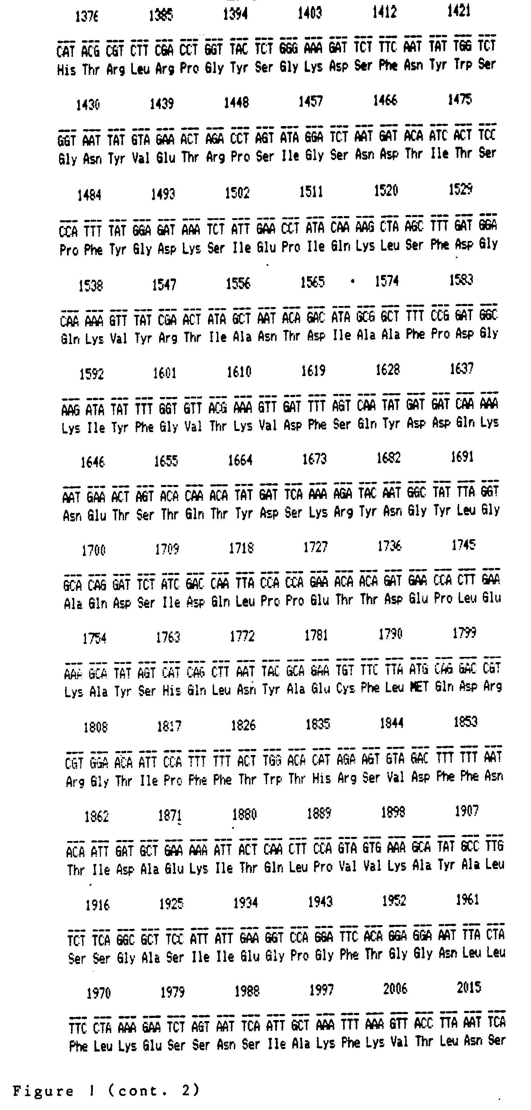

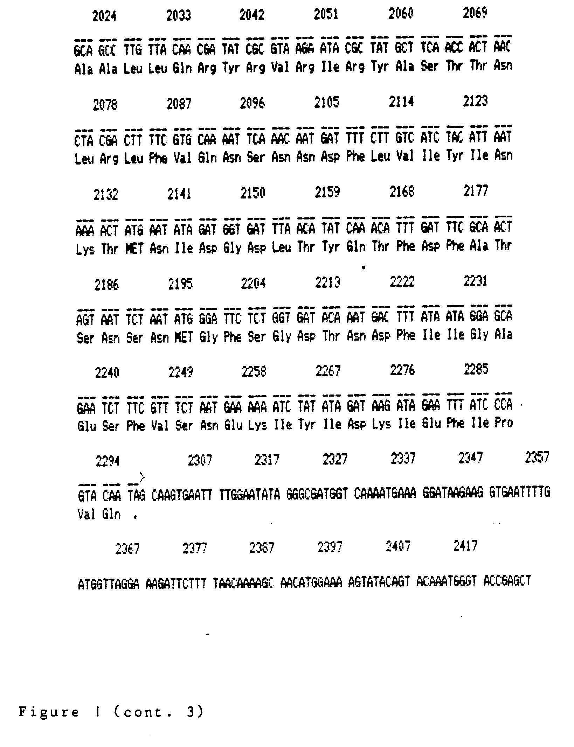

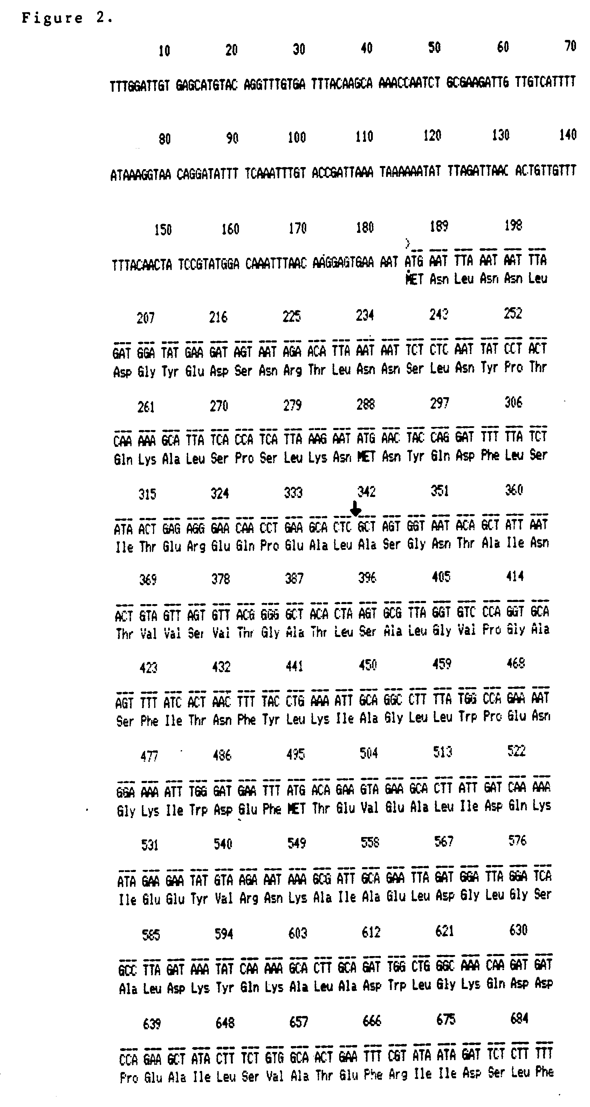

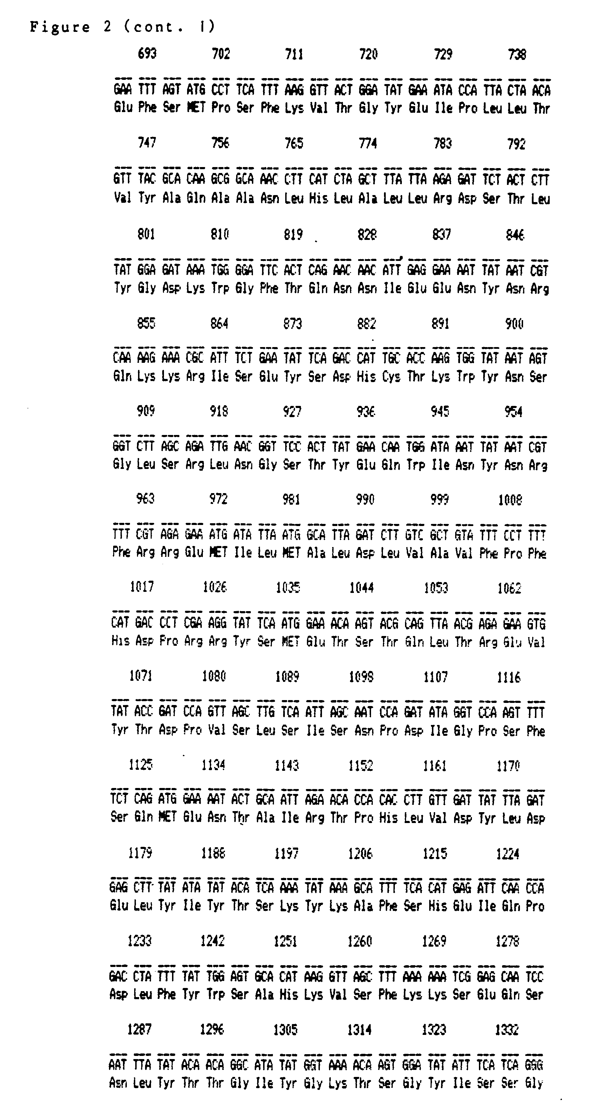

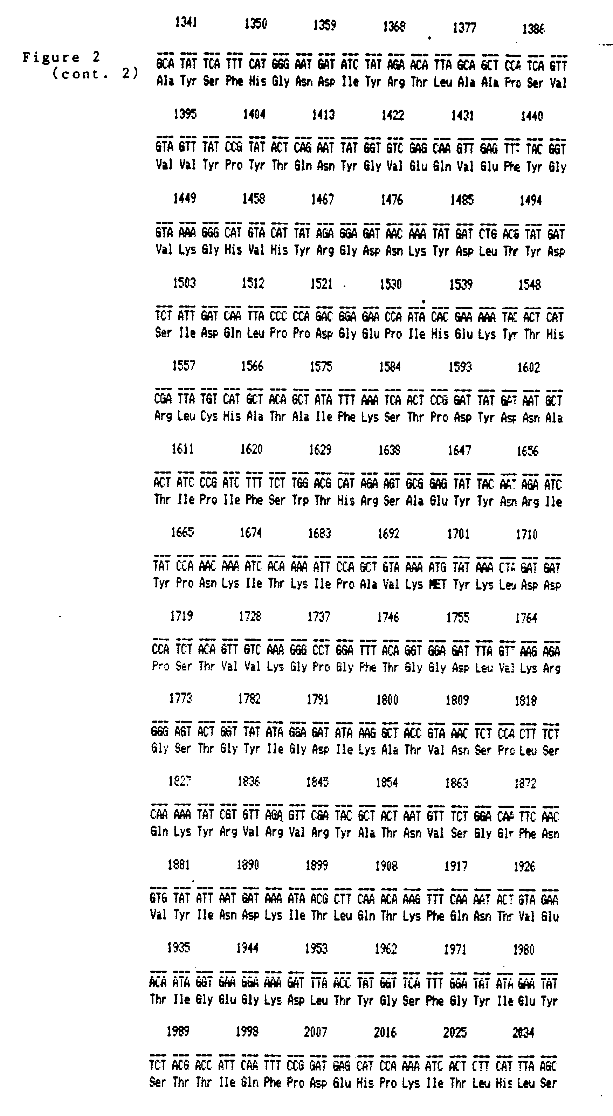

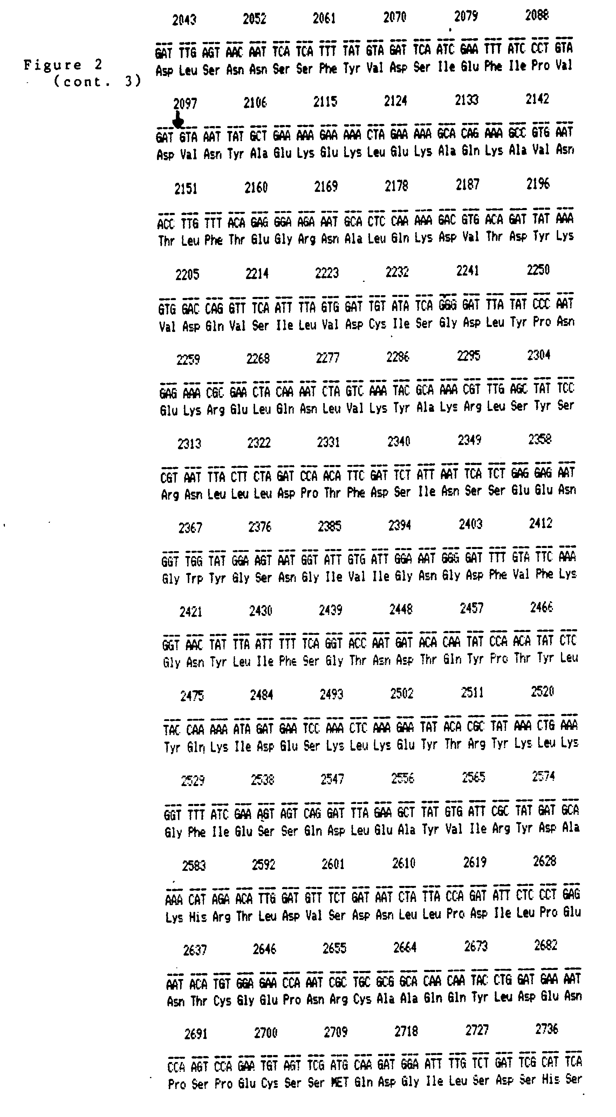

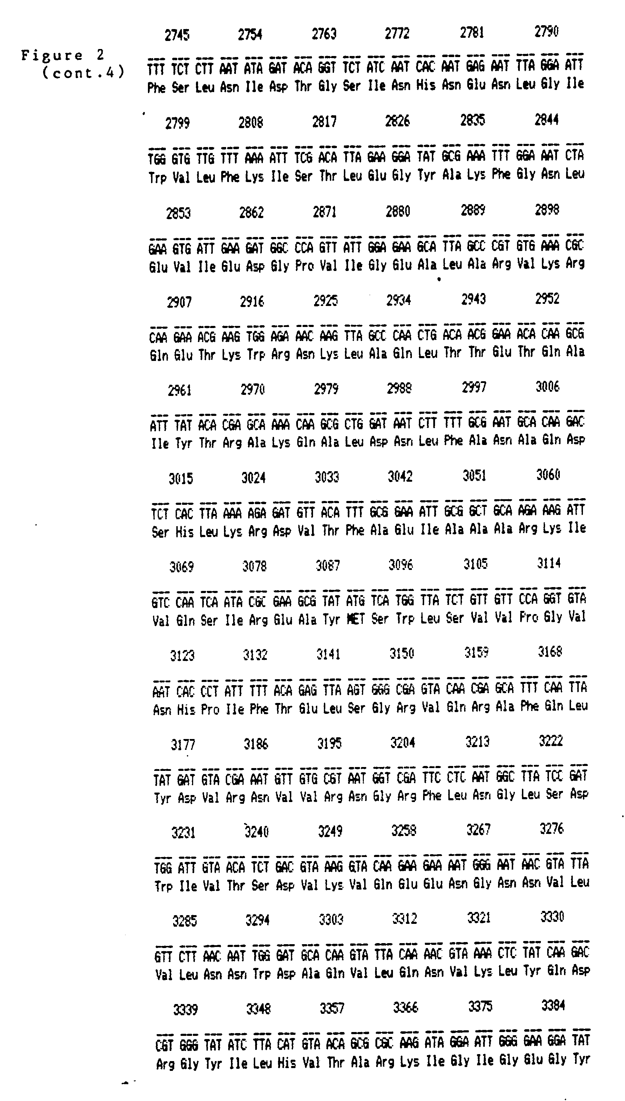

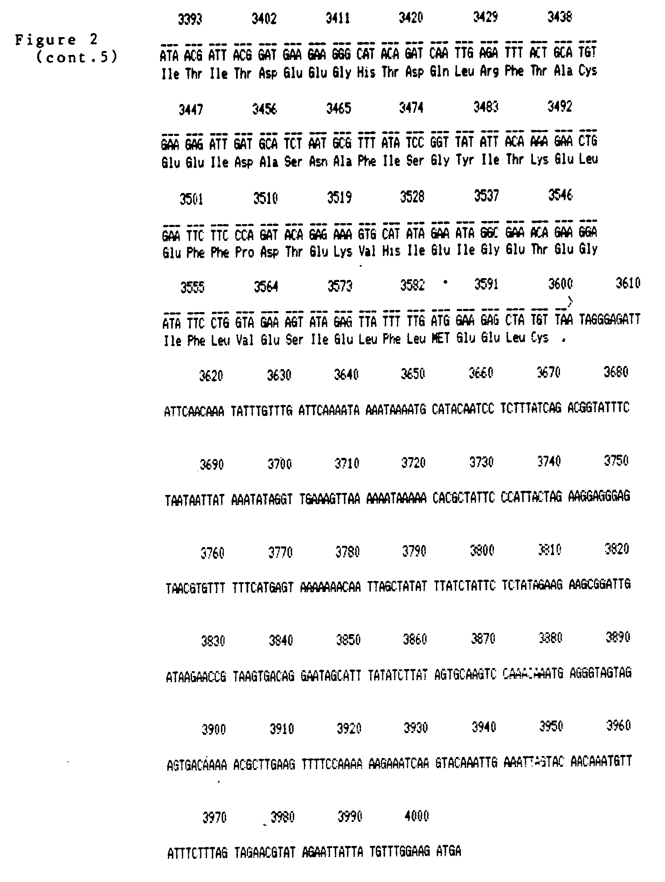

The so-identifed btPGSI208 and btPGSI245 genes can then each be sequenced in a conventional manner (Maxam and Gilbert, 1980) to obtain the DNA sequences shown in Figs. 1 and 2, respectively.

A truncated part of each of the sequenced genes, encoding an insecticidally effective portion of its protoxin and preferably encoding just its toxin, can be made in a conventional manner from the gene after the gene has been sequenced. The aminoacid sequences of the BtPGSI208 and BtPGSI245 protoxins and toxins can be determined from the DNA sequences of their respective btPGSI208 and btPGSI245 genes and truncated btPGSI208 and btPGSI245 genes

The insecticidally effective btPGSI208 or btPGSI245 gene part, encoding an insecticidally effective portion of its respective BtPGSI208 or BtPGSI245 protoxin, can be stably inserted in a conventional manner into the nuclear genome of a single plant cell, and the so-transformed plant cell be used in a conventional manner to produce a transformed plant that is insect-resistant. In this regard, a disarmed Ti-plasmid, containing the insecticidically effective btPGSI208 or btPGSI245 gene part, in Agrobacterium tumefaciens can be used to transform the plant cell, and thereafter, a transformed plant can be regenerated from the transformed plant cell using the procedures described, for example, in European patent publication 0,116,718, PCT publication WO 84/02,913 and European patent application 87/400,544.0 (which are also incorporated herein by reference). The resulting transformed plant can be used in a conventional plant breeding scheme to produce more transformed plants with the same characteristics or to introduce the insecticidally effective btPGSI208 or btPGSI245 gene part in other varieties of the same or related plant species. Seeds, which are obtained from the transformed plants, contain the insecticidally effective btPGSI208 or btPGSI245 gene part as a stable genomic insert.

The insecticidally effective btPGSI208 or btPGSI245 gene part, preferably the truncated btPGSI208 or btPGSI245 gene, is inserted in a plant cell genome so that the inserted part of the gene is downstream of, and under the control of, a promoter which can direct the expression of the gene part in the plant cell. This is preferably accomplished by inserting the btPGSI208 or btPGSI245 chimaeric gene in the plant cell genome. Preferred promoters include: the strong constitutive 35S promoters (the "35S promoters") of the cauliflower mosaic virus of isolates CM 1841 Gardner et al, 1981), CabbB-S (Franck et al, 1980) and CabbB-JI (Hull and Howell, 1987); and the TR1′ promoter and the TR2′ promoter (the "TR1′ promoter" and "TR2′ promoter", respectively) which drive the expression of the 1′ and 2′ genes, respectively, of the T-DNA (Velten et al, 1984). Alternatively, a promoter can be utilized which is not constitutive but rather is specific for one or more tissues or organs of the plant (e.g., leaves and/or roots) whereby the inserted btPGSI208 or btPGSI245 gene part is expressed only in cells of the specific tissue(s) or organ(s). For example, the btPGSI208 or btPGSI245 gene part could be selectively expressed in the leaves of a plant (e.g., potato) by placing the gene part under the control of a light-inducible promoter such as the promoter of the ribulose-1,5-bisphosphate carboxylase small subunit gene of the plant itself or of another plant such as pea as disclosed in U.S. patent application 821,582 and European patent application 86/300,291.1. Another alternative is to use a promoter whose expression is inducible (e.g., by temperature or chemical factors).

The insecticidally effective btPGSI208 or btPGSI245 gene part is inserted in the plant genome so that the inserted part of the gene is upstream of suitable polyadenylation and transcription termination signals. This is preferably accomplished by inserting the btPGSI208 or btPGSI245 chimaeric gene in the plant cell genome. Preferred polyadenylation and transcription termination signals include those of the octopine synthase gene (Gielen et al, 1984) and the T-DNA gene 7 (Velten and Schell, 1985), which act as 3′-untranslated DNA sequences in transformed plant cells.

It is preferred that the inserted insecticidally effective btPGSI208 or btPGSI245 gene part be in the same transcriptional unit as, and under the control of, the same promoter as a selectable marker gene. This is preferably accomplished by inserting a btPGSI208 or btPGSI245 chimaeric gene, containing the marker

4

gene, in the plant cell genome. Any conventional marker gene can be utilized, the expression of which can be used to select transformed plant cells. An example of a suitable selectable marker gene is an antibiotic resistance gene such as the neo gene coding for kanamycin resistance (Reiss et al, 1984; European patent application 87/400,544.0; U.S. patent application 821,582; European patent application 86/300,291.1). Thereby, the insecticidally effective btPGSI208 or btPGSI245 gene part and the marker gene (e.g., the btPGSI208-neo or btPGSI245-neo hybrid gene) are expressed in a transformed plant as a fusion protein (U.S. patent application 821,582; European patent application 86/300,291.1; Vaeck et al, 1987).

Each of the BtPGSI208 and BtPGSI245 strains can be fermented by conventional methods (Dulmage, 1981) to provide high yields of cells. Under appropriate conditions which are well understood (Dulmage, 1981), the BtPGSI208 and BtPGSI245 strains each sporulate to provide their respective BtPGSI208 and BtPGSI245 crystal proteins in high yields.

An insecticide composition of this invention can be formulated in a conventional manner using the BtPGSI208 or BtPGSI245 strain or preferably their respective crystals, crystal proteins, protoxin, toxin and/or insecticidally effective portions of their respective protoxin, as active ingredient(s), together with suitable carriers, diluents, emulsifiers and/or dispersants. This insecticide composition can be formulated as a wettable powder, pellets, granules or a dust or as a liquid formulation with aqueous or non-aqueous solvents as a foam, gel, suspension, concentrate, etc. The concentration of the BtPGSI208 or BtPGSI245 strain, crystals, crystal proteins, protoxin, toxin and/or protoxin portions in such a composition will depend upon the nature of the formulation and its intended mode of use. Generally, an insecticide composition of this invention can be used to protect a potato field for 2 to 4 weeks against Coleoptera with each application of the composition. For more extended protection (e.g., for a whole growing season), additional amounts of the composition should be applied periodically.

The following examples illustrate the invention. The figures, referred to in the examples, are as follows :

Figure 1 - DNA sequence of the btPGSI208 gene. The derived aminoacid sequence of the encoded BtPGSI208 protoxin is presented beneath this sequence. The arrow separates the N-terminal 57 aminoacids from the C-terminal portions encoding the BtPGSI208 toxin.

Figure 2 - DNA sequence of the btPGSI245 gene. The derived aminoacid sequence of the encoded BtPGSI245 protoxin is presented beneath this sequence. The arrows delineate the BtPGSI245 toxin between aminoacids 54 and 638 of the BtPGSI245 protoxin.

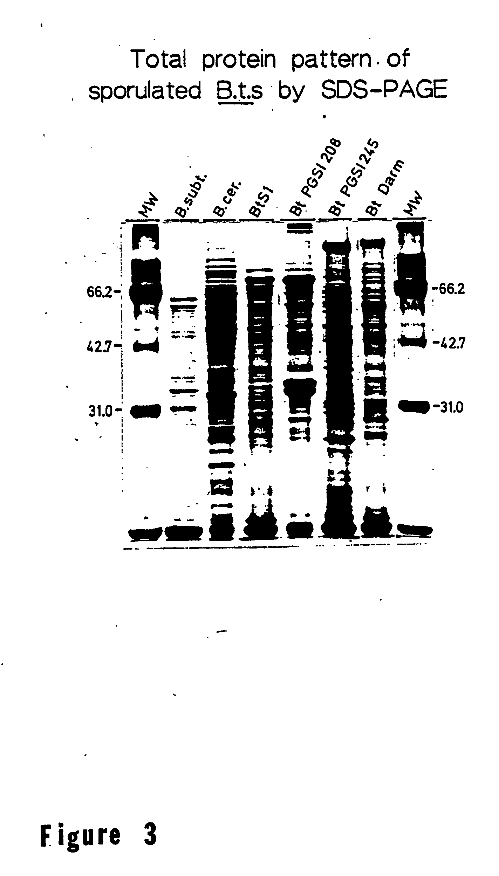

Figure 3 - Total protein patterns by SDS-PAGE of sporulated BtPGSI208 and BtPGSI245 and other Bacillus cultures. Among the comparison strains, B. subt. is Bacillus subtilis, B. cer. is Bacillus cereus, and Bt Darm is Bacillus thuringiensis subsp. darmstadiensis. These comparison strains were obtained from the sources set forth in Table 1, hereinafter. "MW" designates molecular weight markers.

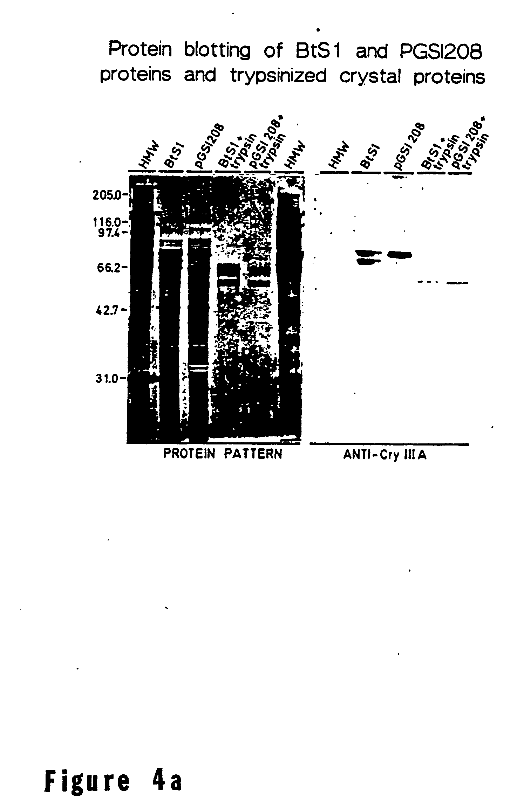

Figure 4A- Protein blotting of total proteins and trypsinized crystal proteins from strains BtS1 and BtPGSI208. Total protein patterns were stained with Indian ink, while crystal proteins were visualized with an antiserum against Bt13 toxin ("anti-CryIIIA"). "HMW" designates molecular weight markers.

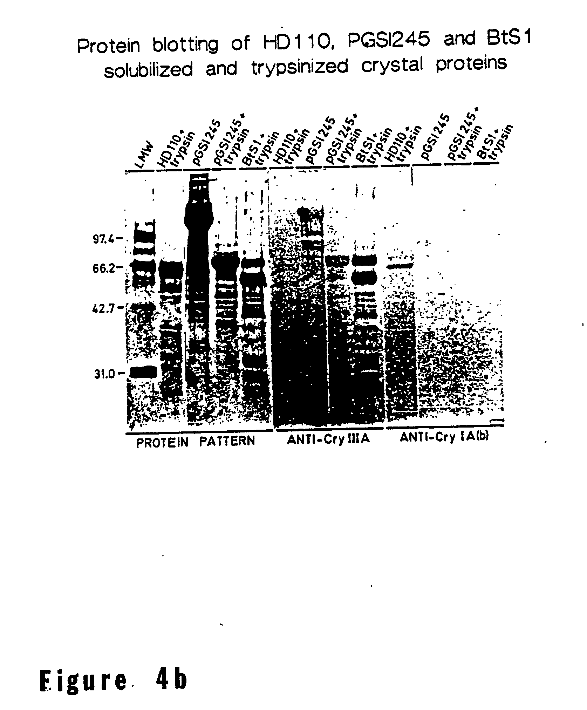

Figure 4B- Protein blotting of total proteins and trypsinized crystal proteins from strains BtS1, BtPGSI245 and Bt HD-110. Total protein patterns were probed for their immunoreactivity with an antiserum against Bt13 toxin ("anti-CryIIIA") and an antiserum against Bt2 protoxin ("anti-CryIA(b)"). "LMW" designates molecular weight markers. The comparison strain, HD-110, was Bt HD-110, obtained from Dr. H. Dulmage, Cotton Insect Laboratories, U.S.D.A., Brownsville, Texas, U.S.A.

Unless otherwise stated in the Examples, all procedures for making and manipulating recombinant DNA are carried out by the standardized procedures described in Maniatis et al, Molecular Cloning - A laboratory Manual, Cold Spring Harbor Laboratory (1982).

Example 1 : Characterization of the BtPGSI208 and BtPGSI245 strains

The BtPGSI208 strain was isolated from grain dust sampled in Belgium and was deposited at the DSM on January 19, 1989 under accession No. 5131.

The BtPGSI245 strain was isolated from cow dung sampled in the United States and was deposited at the DSM on January 19, 1989 under accession No. 5132.

Each strain can be cultivated on conventional standard media, preferably LB medium (Bacto-tryptone 10 g/l, yeast extract 5 g/l, NaCl 10 g/l and agar 15 g/l), preferably at 28° C. For long term storage, it is preferred to use LB liquid medium containing 50% glycerol at -70° C or lyophilization. For sporulation, the use of T$_3$ medium (tryptone 3 g/l, tryptose 2 g/l, yeast extract 1.5 g/l, 5 mg MnCl$_2$, 0.05 M Na$_2$PO$_4$, pH 6.8 and 1.5% agar) is preferred for 24 hours at 28° C, followed by storage at 4° C. During its vegetative phase, each of the BtPGSI208 and BtPGSI245 strains can also grow under facultative anaerobic conditions, but sporulation only occurs under aerobic conditions.

Sterilization of each strain occurs by autoclave treatment at 120°C (1 bar pressure) for 20 minutes. Such treatment totally inactivates the spores and the crystalline BtPGSI208 and BtPGSI245 protoxins. UV radiation (254 nm) inactivates the spores but not the protoxins.

After cultivating on Nutrient Agar ("NA", Difco Laboratories, Detroit, MI, USA) for one day, colonies of each of the BtPGSI208 and BtPGSI245 strains form opaque white colonies with irregular edges. Cells of each strain (Gram positive rods of 1.7-2.4 x 5.6-7.7 μm) sporulate after three days cultivation at 28°C on NA.

The crystal proteins produced during sporulation are packaged in flat rhomboid crystals in the BtPGSI208 strain and in bipyramidal crystals in the BtPGSI245 strain. Both of these crystal forms are clearly different from the flat square crystals of the BtS1 strain (from DSM under accession no. 4288) or Bt tenebrionis (from DSM under accession no. 2803).

For the biochemical characterization of the two strains, the following tests were carried out using well known methods as described for example by Sneath et al (1986). Growth was observed in Nutrient Broth ("NB", Difco) supplemented with 2 and 5% NaCl. No growth of the BtPGSI208 strain and only weak growth of the BtPGSI245 strain were observed in the presence of 7% NaCl. Neither strain grew in medium supplemented with 10% NaCl. The BtPGSI208 and BtPGSI245 strains grew well on NA at 20, 28 and 37°C, but not at 4, 10 (although the BtPGSI245 strain grew slowly at this temperature), 50 and 60°C. Both strains grew in NB at pH = 5, pH = 6 and pH = 7 and on NB containing 100 units of lysozyme (Sigma Chemical Company, St Louis, MO, USA) per ml of NB. Growth on NA under anaerobiosis was very weak.

Metabolic characteristics of the two strains were determined using API-20E test strips (API Systems S.A., Montalieu-Vercieu, France). The results of these assays are shown in Table 1, below.

Table 1

| Metabolic characteristics of the BtPGSI208 and BtPGSI245 strains as compared with other Bacillus strains | | | | | | |
|---|---|---|---|---|---|---|
| (+ = positive reaction ; - = negative reaction ; w = weak reaction ; nd = not determined). | | | | | | |
| Activity | Bt PGSI 208 | Bt PGSI 245 | BTS1 | BTEN | BDAR | BCER | BSUB |
| ONPG | - | - | - | - | - | - | + |
| ADH | - | + | + | + | + | + | - |
| LDC | - | - | - | - | - | - | - |
| ODC | - | - | - | - | - | - | - |
| CIT | - | - | - | - | - | - | - |
| H2S | - | - | - | - | - | - | - |
| URE | - | - | - | - | - | - | - |
| TDA | - | - | - | + | w | + | + |
| IND | - | - | - | - | - | - | - |
| VP | - | - | - | w | w | w | + |
| GEL | - | + | - | + | + | + | + |
| OX | + | + | + | + | + | + | + |
| NO2 | + | + | + | + | + | + | nd |
| N2 | - | - | - | - | - | - | nd |

ONPG = β-galactosidase activity.
DH = arginine dihydrolase activity.
LDC = lysine decar oxylase activity.
ODC = ornithine decarboxylase activity.
CIT = use of citrate as sole carbon source.
H2S = H2S formation from thiosulphate.
URE = urease activity.
TDA = tryptophan deaminase activity.
IND = indol formation from tryptophan.

VP = acetoin formation from sodium pyruvate.

GEL = gelatin liquefaction.

OX = oxidase activity.

NO2 = nitrate reduction to nitrite.

N2 = $N_2$ gas production from nitrate.

BTS1 = Bacillus thuringiensis BtS1 from DSM under accession no. 4288.

BTEN = Bacillus thuringiensis subsp. tenebrionis from DSM under accession no. 2803.

BDAR = Bacillus thuringiensis subsp. darmstadiensis from (Institut für Landwirtschaftliche Bacteriologie und Gärungsbiologie der Eidgenössiche Technische Hochschüle, Zürich, Switzerland ("LBG"), under accession no. 4447

BCER = Bacillus cereus from Laboratorium voor Microbiologie, Gent, Belgium ("LMG"), under accession no. 2098.

BSUB = Bacillus subtilis from Agricultural Research Culture Collection, Peoria, Illinois, USA, under accession no. NRRL B-237.

Both strains were found to rapidly decompose casein in skim-milk agar and to deaminate phenylalanine in tests described by Sneath et al (1986).

Acid production from different sugars by the two strains was determined using API-50CHB test strips (API Systems SA). The results are shown in Table 2, below.

Table 2: Acid production by the BtPGSI208 and BtPGSI245 strains as compared with other bacilli (+ = positive reaction ; − = negative reaction ; w = weak reaction).

| Substrate : | Bt PGSI 208 | Bt PGSI 245 | BtS1 | BTEN | BDAR | BCER | BSUB |
|---|---|---|---|---|---|---|---|
| Control | − | − | − | − | − | − | − |
| Glycerol | − | w | + | + | + | + | + |
| Erythritol | − | − | − | − | − | − | − |
| D-arabinose | − | − | − | − | − | − | − |
| L-arabinose | − | − | − | − | − | − | + |
| Ribose | + | + | + | + | + | + | + |
| D-Xylose | − | − | − | − | − | − | + |
| L-Xylose | − | − | − | − | − | − | − |
| Adonitol | − | − | − | − | − | − | − |
| B Methyl-xyloside | − | − | − | − | − | − | − |
| Galactose | − | − | − | − | − | − | + |
| D-Glucose | + | + | + | + | + | + | + |
| D-Fructose | + | + | + | + | + | + | + |
| D-Mannose | − | + | + | + | − | − | + |
| L-Sorbose | − | − | − | − | − | − | − |
| Rhamnose | − | − | − | − | − | − | − |
| Dulcitol | − | − | − | − | − | − | − |
| Inositol | − | − | − | − | − | − | + |
| Mannitol | − | − | − | − | − | − | + |
| Sorbitol | − | − | − | − | − | − | + |
| α-Methyl-D-mannoside | − | − | − | − | − | − | − |
| α-Methyl-D-glucoside | − | − | − | − | − | − | + |
| N-Acetylglucosamide | + | + | + | + | + | + | − |
| Amygdaline | − | − | − | − | − | − | + |
| Arbutine | + | + | + | + | + | − | + |
| Esculine | + | + | + | + | + | + | + |
| Salicine | + | w | − | − | − | − | + |
| Cellobiose | − | w | − | − | − | − | + |
| Maltose | + | + | + | + | + | + | + |
| Lactose | − | − | − | − | − | − | + |
| Melibiose | − | − | − | − | − | − | + |
| Saccharose | + | + | + | + | − | + | + |
| Trehalose | + | + | + | + | + | + | + |
| Inuline | − | − | − | − | − | − | + |

8

## Table 2 (Continued)

| Substrate : | Bt PGSI 208 | Bt PGSI 245 | BtS1 | BTEN | BDAR | BCER | BSUB |
|---|---|---|---|---|---|---|---|
| Melizitose | - | - | - | - | - | - | - |
| D-Raffinose | - | - | - | - | - | - | + |
| Starch | + | + | + | + | + | + | + |
| Glycogen | + | + | + | + | + | + | + |
| Xylitol | - | - | - | - | - | - | - |
| B Gentiobiose | - | - | - | - | - | - | - |
| D-Turanose | - | - | - | - | - | - | + |
| D-Lyxose | - | - | - | - | - | - | - |
| D-Tagatose | - | - | - | - | - | - | - |
| D-Fucose | - | - | - | - | - | - | - |
| L-Fucose | - | - | -. | - | - | - | - |
| D-Arabitol | - | - | - | - | - | - | - |
| L-Arabitol | - | - | - | - | - | - | - |
| Gluconate | - | - | - | - | - | - | - |
| 2 Ketogluconate | - | - | - | - | - | - | - |
| 5 Ketogluconate | - | - | - | - | - | - | - |

Sensitivity of the two strains towards different antibiotics was tested using Oxoid Susceptibility Test Discs on Oxoid Isosensitest agar ("CM471" of Oxoid Ltd., Basingstoke, Hampshire, England). The results are shown in Table 3, below.

Table 3

| Antibiotic sensitivity as shown by the diameters (in mm) of inhibition zones observed after 24 hours on antibiotic-containing agar, seeded with different bacilli | | | | | | | | |
|---|---|---|---|---|---|---|---|---|
| (R = resistant colonies or no growth detected). | | | | | | | | |
| Antibiotic | amount/disc | Bt PGSI 208 | Bt PGSI 245 | BtS1 | BTEN | BDAR | BCER | BSUB |
| Chloramphenicol | 30 ug | 25/R | 17 | 19/R | 20 | 22 | 28 | 33 |
| Bacitracin | 10 i.u | 11 | 10 | 8 | 7 | 14 | 18 | 7 |
| Gentamycin | 10 ug | 26 | 20 | 21 | 20 | 28 | 9 | 25/R |
| Neomycin | 30 ug | 24 | 20/R | 13/R | 13 | 26 | 10 | 20 |
| Tetracyclin | 30 ug | 14/R | 10 | 17/R | 16/R | 10/R | 21 | 22 |
| Carbenicillin | 100 ug | 8 | 11 | 0 | 0 | 10 | 0 | 19 |
| Rifampicin | 2 ug | 12 | 13 | 0 | 8 | 8 | 19 | 26 |
| Penicillin G | 10 i.u | 7 | 8 | 0 | 0 | 0 | 14/R | 14 |
| Streptomycin | 10 ug | 25/R | 15 | 16 | 17 | 20 | 14 | 0 |
| Spectinomycin | 10 ug | 0 | 0 | 0 | 0 | 0 | 12/R | 0 |
| Kanamycin | 30 ug | 20/R | 21/R | 0 | 0 | 24 | 15 | 24 |
| Nalidixic acid | 30 ug | 25/R | 23/R | 18/R | 25/R | 30/R | 7 | 19 |
| Sulphamethoxazole | 25 ug | 0 | 0 | 0 | 0 | 0 | 0 | 0 |
| Trimethoprim | 2.5 ug | 0 | 0 | 0 | 0 | 0 | 0 | 26 |
| Ampicillin | 10 u | 7 | 9/R | 0 | 0 | 0 | 15/R | 18 |

The enzyme spectra of the BtPGSI208 and BtPGSI245 strains were determined using the extended API-ZYM strips (API Systems S.A.). The results are shown in Table 4, below. Esterase-, peptidase- (AP1, AP2, AP3, AP4, AP5 and AP6 test strips) and osidase-test strips were inoculated with 50 µl cell suspension

($10^7$ cfu/ml). The osidase reaction was revealed after 4 hours incubation (28°C) with 25 µl 0.1N NaOH. All other reactions were with 25 µl ZYM A and ZYM B reagent (API no. 7048).

Table 4

| Enzymatic spectra of the BtPGSI208 and BtPGSI245 strains as compared to two other Bt strains | | | | |
|---|---|---|---|---|
| (0 = no substrate used; 1, 2, 3, 4, 5 = 5, 10, 20, 30 and ≥ 40 nanomoles of substrate hydrolysed respectively). | | | | |
| Substrate | Bt PGSI 208 | Bt PGSI 245 | BTS1 | BDAR |
| Esterases. | | | | |
| 2-naphtyl-valerate | 4 | 4 | 2 | 4 |
| 2-naphtyl-caproate | 5 | 5 | 5 | 5 |
| 2-naphtyl-caprylate | 5 | 5 | 5 | 5 |
| 2-naphtyl-nonanoate | 5 | 5 | 4 | 5 |
| 2-naphtyl-caprate | 5 | 3 | 3 | 5 |
| 2-naphtyl-laurate | 2 | 2 | 1 | 2 |
| 2-naphtyl-myristate | 1 | 2 | 1 | 1 |
| 2-naphtyl-palmitate | 0 | 0 | 1 | 0 |
| 2-naphtyl-stearate | 2 | 1 | 2 | 2 |
| Peptidases. | | | | |
| L-pyrrolidonyl-β-naphtylamide | 0 | 5 | 5 | 5 |
| Glycyl-β-naphtylamide | 0 | 0 | 0 | 0 |
| L-glutamyl-β-naphtylamide | 0 | 0 | 0 | 0 |
| L-leucyl-glycyl-β-naphtylamide | 0 | 1 | 0 | 0 |
| L-seryl-L-tyrosyl-β-naphtylamide | 4 | 5 | 5 | 5 |
| L-glutamine-β-naphtylamide | 1 | 5 | 4 | 5 |
| L-glutamyl-β-naphtylamide | 0 | 3 | 3 | 3 |
| Osidases. | | | | |
| Paranitrophenol-D-galactopyranoside | 0 | 0 | 0 | 0 |
| Paranitrophenol-βD-galactopyranoside | 0 | 0 | 0 | 0 |
| Paranitrophenol-aD-glucopyranoside | 5 | 5 | 5 | 5 |
| Paranitrophenol-βD-glucopyranoside | 0 | 2 | 0 | 0 |
| Paranitrophenol-a-maltoside | 2 | 4 | 3 | 5 |
| Paranitrophenol-β-maltoside | 0 | 0 | 0 | 0 |
| Paranitrophenol-N-acetyl-βD-glucosamidine | 3 | 5 | 5 | 5 |
| Paranitrophenol-βD-xylapyranoside | 0 | 0 | 0 | 0 |

Example 2: Characteristics of the BtPGSI208 and BtPGSI245 crystals

The BtPGSI208 and BtPGSI245 strains were grown for 48 to 72 hours at 28°C on $T_3$ medium. After sporulation, the spores and crystals were harvested in phosphate buffered saline solution ("PBS" from Oxoid Ltd.) by scraping with a Trihalski spatula. The resulting aqueous spore-crystal suspensions were centrifuged, and the pellets were resuspended and incubated overnight in aqueous solutions containing 50mM $Na_2CO_3$ and 5mM dithiotreitol ("DTT") at pH 10. After centrifugation, the supernatants were recovered containing the respective crystal proteins.

The BtPGSI208 protoxin and toxin, as well as the BtPGSI245 toxin, react only with a polyclonal antiserum raised against the Bt13 toxin as shown in Fig. 4. In contrast, the BtPGSI245 protoxin reacts with polyclonal antisera against the Bt13 toxin and the Bt2 protoxin (U.S. patent application 821,582; European

10

patent application 86/300,291.1) as shown in Fig. 4.

The total protein patterns of the BtPGSI208 and BtPGSI245 strains, compared to other Bacillus strains, are shown in Fig. 3. For this comparison, the crystal proteins of each strain were analyzed on a 12.5% SDS-PAGE gel (Laemmli, 1970) and stained with Coomassie brilliant blue R-250 according to Lambert et al (1987). The crystal proteins were dissolved by exposing the spore-crystal mixtures overnight at 37°C to 50 mM $Na_2CO_3$, pH 10, 5 mM DTT. Solubilized crystal proteins were digested by adjusting the pH to 9.0 with 0.5 M HCl and by trypsinization (1 μg bovine trypsin/25 μg protein). Trypsin digestion of the BtPGSI208 and BtPGSI245 crystal proteins was performed at 37°C overnight and revealed the presence of tryptic fragments of 68 kDa (Fig. 4). Immunoblotting experiments, performed according to Peferoen (1988) with polyclonal antisera raised against the Bt13 toxin and the Bt2 protoxin demonstrated, in Figs. 4A and B, that the BtPGSI208 and BtPGSI245 protoxins and toxins are immunologically related to the Bt13 toxin. In addition, the BtPGSI245 protoxin was also shown, in Fig. 4B, to be immunologically related to the Bt2 protoxin. After blotting, the proteins were stained with Indian ink (Sutherland and Skerritt, 1986) to show both immunoreactive and non-immunoreactive proteins (Fig. 4).

Example 3: Insecticidal activity of the BtPGSI208 and BtPGSI245 crystal proteins

As in Example 2, both strains were grown for 48 to 72 hrs at 28°C on T₃ medium. After sporulation, the spores and crystals were harvested in PBS with a Trihalski spatula. The resulting spore-crystal suspensions were centrifuged, and the pellets were resuspended and incubated overnight in aqueous $Na_2CO_3$ and DTT solutions as described in Example 2. After centrifugation, the supernatants were recovered, and their contents of the respective crystal proteins of the two strains were determined.

Potato leaves were dipped in aqueous dilutions of the supernatant solutions and then air dried for two hours. Colorado potato beetle larvae of the second instar were placed on the treated leaves, and mortality of the larvae was measured after three days. These results were compared with the mortality of larvae fed leaves treated with solubilized crystals of Bt HD-1 ("Bt kurstaki Dipel" from Abbott Laboratories, Abbott Park, North Chicago, Ill., USA) as a control. LC₅₀, expressed as ug of solubilized crystals/ml, was calculated by Probit analysis (Finney, 1971). The results are summarized in Table 5, below.

Table 5

| Strain | LC50 | FL95min | FL95max | Slope |
|-----------|------|---------|---------|-------|
| BtPGSI208 | 5.0 | 3.5 | 7.3 | 2.4 |
| BtPGSI245 | 25.1 | 14.7 | 43.3 | 1.5 |
| Control | >500 | - | - | - |

Example 4: Identification and cloning of the btPGSI208 gene

The BtPGSI208 protoxin from the BtPGSI208 strain was detected by ELISA (Engvall and Pesce, 1978) with a polyclonal antiserum against the Bt13 coleoptera toxin (Höfte et al, 1987). The btPGSI208 gene was identified in the BtPGSI208 strain by preparing total DNA of the BtPGSI208 strain and then digesting the DNA with the restriction enzymes HindIII, EcoRI and ClaI. The so-digested DNA was analyzed by Southern blotting, probing with a nick-translated 2.9 kb HindIII fragment from the genome of the BtS1 strain (European patent application 88/402,115.5) containing the bt13 gene. After hybridization with the probe, the blot was washed under low stringency conditions (2XSSC, 0.1%SDS at 68°C for 2x15 min), showing the presence of the btPGSI208 gene, related to the bt13 gene. The hybridization pattern with the probe also showed that the btPGSI208 gene was clearly different from the bt13 gene.

In order to isolate the btPGSI208 gene, total DNA was prepared from the BtPGSI208 strain. The total DNA preparation was partially digested with Sau3A and was size-fractionated on a sucrose gradient. Fractions containing DNA between 5 kb and 10 kb were ligated to the BglII-digested and bovine alkaline phosphatase ("BAP")-treated cloning vector pEcor251 (Deposited at the DSM on July 13, 1988 under accession number DSM 4711). Recombinant E. coli clones containing the vector were then screened with the 2.9 kb HindIII DNA fragment containing the bt13 gene, as a probe, to identify DNA fragments of clones containing the btPGSI208 gene. The so-identified DNA fragments were then sequenced according to Maxam and Gilbert (1980).

Based on the analysis of the DNA sequence of the btPGSI208 gene, the gene is cut with an appropriate restriction enzyme to give the truncated btPGSI208 gene, encoding the BtPGSI208 toxin of about 67 kDa.

Example 5: Identification and cloning of the btPGSI245 gene

The BtPGSI245 protoxin from the BtPGSI245 strain was detected by ELISA (Engvall and Pesce, 1978) with a polyclonal antiserum directed against the Bt13 coleoptera toxin. Colony hybridization of the BtPGSI245 strain and Southern blotting of the BtPGSI245 total DNA, that had been probed with the 2.9 kb HindIII DNA fragment containing the bt13 gene and washed under the previously described low stringency conditions, revealed no hybridizing DNA.

In order to isolate the btPGSI245 gene, total DNA from the BtPGSI245 strain was prepared and partially digested with Sau3A. The digested DNA was size fractionated on a sucrose gradient and fragments ranging from 5 kb to 10 kb were ligated to the BamHI,-digested and BAP-treated cloning vector pUC18 (Yannisch-Perron et al, 1985). Recombinant clones containing the vector were then screened by colony immunoprobing (French et al, 1986) with an antiserum against the Bt13 toxin. Positive colonies were purified, and total protein preparations were again analyzed by immunoblotting with the antiserum against the Bt13 toxin. DNA fragments, containing the BtPGSI245 gene, from clones expressing the BtPGSI245 protoxin, were then sequenced according to Maxam and Gilbert (1980).

Based on the analysis of the DNA sequence of the btPGSI245 gene, the gene is cut with an appropriate restriction enzyme to give the truncated btPGSI245 gene, encoding the BtPGSI245 toxin of about 66 kDa.

Example 6: Construction of a btPGSI208-neo hybrid gene and a btPGSI245-neo hybrid gene

Following the procedure of European patent application 88/402,115.5, the truncated btPGSI208 and btPGSI245 genes from Examples 4 and 5, respectively, are each fused to the neo gene to form the corresponding hybrid gene.

Example 7: Insertion of the btPGSI208 and btPGSI245 genes, the truncated btPGSI208 and btPGSI245 genes and the btPGSI208-neo and btPGSI245-neo hybrid genes in E. coli and insertion of the truncated btPGSI208 and btPGSI245 genes and the btPGSI208-neo and btPGSI245-neo hybrid genes in potato plants

The btPGSI208 and btPGSI245 genes and the truncated btPGSI208 and btPGSI245 genes from Examples 4 and 5 and the btPGSI208-neo and btPGSI245-neo hybrid genes from Example 6 are each inserted into, and expressed by, different E. coli in a conventional manner, using E. coli expression vectors as described by Botterman and Zabeau (1987).

Using the procedures described in U.S. patent application 821,582 and European patent applications 86/300,291.1 and 88/402,115.5, the truncated btPGSI208 and btPGSI245 genes and the btPGSI208-neo and btPGSI245-neo hybrid genes are inserted into intermediate plant expression vectors. To provide major expression in plants, the hybrid genes and truncated genes are placed under the control of strong constitutive promoters such as the Cabb-JI 35S promotor (Hull and Howell, 1987) or the TR2' promoter (Velten et al, 1984) and are fused to transcription termination and polyadenylation signals such as those of the octopine synthase gene (Gielen et al, 1984).

Using standard procedures (Deblaere et al, 1985), the intermediate plant expression vectors, containing the truncated btPGSI208 and btPGSI245 genes and the btPGSI208-neo and btPGSI245-neo hybrid genes, are transferred into the Agrobacterium strain C 58 C1 Rif[R] (US patent application 821,582; European patent application 86/300,291.1) carrying the disarmed Ti-plasmid pGV2260 (Vaeck et al, 1987). Selection for spectinomycin resistance yields cointegrated plasmids, consisting of pGV2260 and the respective intermediate plant expression vectors. Each of these recombinant Agrobacterium strains is then used to transform different potato plant cells so that the truncated btPGSI208 gene, the truncated btPGSI245 gene, the btPGSI208-neo hybrid gene and the btPGSI245-neo hybrid gene are contained in, and expressed by, different cells.

Example 8: Expression of the truncated btPGSI208 and btPGSI245 genes and the btPGSI208-neo and btPGSI245-neo hybrid genes in potato plants

The insecticidal activity against Coleoptera of the expression products of the truncated btPGSI208 and btPGSI245 genes and the btPGSI208-neo and btPGSI245-neo hybrid genes in leaves of transformed potato plants, generated from the transformed potato plant cells of Example 7, is evaluated by recording the growth rate and mortality of Leptinotarsa decemlineata larvae fed on these leaves. These results are compared with the growth rate of larvae fed leaves from untransformed potato plants. Toxicity assays are performed as described in European Patent application 88/402,115.5, U.S. patent application 821,582 and European patent application 86/300,291.1. A significantly higher mortality rate is obtained among larvae fed on leaves of transformed potato plants containing the truncated btPGSI208 gene, the truncated btPGSI245 gene, the btPGSI208-neo hybrid gene or the btPGSI245-neo hybrid gene than among larvae fed the leaves of untransformed plants.

Needless to say, this invention is not limited to the BtPGSI208 (DSM 5131) strain and the BtPGSI245 (DSM 5132) strain. Rather, the invention also includes any mutant or variant of the BtPGSI208 or BtPGSI245 strain which produces crystals, crystal proteins, protoxin or toxin having substantially the same properties as the BtPGSI208 or BtPGSI245 crystals, crystal proteins, protoxin or toxin. In this regard, variants of the BtPGSI208 and BtPGSI245 strains include variants whose total protein pattern is substantially the same as the protein pattern of either the BtPGSI208 strain or the BtPGSI245 strain as shown in Fig. 3.

This invention also is not limited to potato plants transformed with the truncated btPGSI208 or btPGSI245 gene. It also includes any plant, such as tomato, tobacco, rapeseed, alfalfa, sunflowers, cotton, corn, soybeans, potato, brassicas, sugar beets and other vegetables, transformed with an insecticidally effective part of the btPGSI208 or btPGSI245 gene.

Nor is this invention limited to the use of Agrobacterium tumefaciens Ti-plasmids for transforming plant cells with an insecticidally effective btPGSI208 or btPGSI245 gene part. Other known techniques for plant cell transformations, such as by means of liposomes, by electroporation or by vector systems based on plant viruses or pollen, can be used for transforming monocotyledons and dicotyledons with such a gene part.

Furthermore, DNA sequences other than those of an insecticidally effective btPGSI208 or btPGSI245 gene part can be used for transforming plants. In this regard, the DNA sequence of either gene part can be modified by: 1) replacing some codons with others that code either for the same amino acids or for other amino acids; and/or 2) deleting or adding some codons; provided that such modifications do not substantially alter the properties of the encoded, insecticidally effective portion of the BtPGSI208 or BtPGSI245 protoxin.

Also, other DNA recombinants containing the aforementioned DNA sequences in association with other foreign DNA, particularly the DNA of vectors suitable for transforming plants and microorganisms other than E. coli, are encompassed by this invention. In this regard, this invention is not limited to the specific plasmids containing the btPGSI208 and btPGSI245 genes, or parts thereof, that were heretofore described, but rather, this invention encompasses any DNA recombinants containing a DNA sequence that is their equivalent. Further, the invention relates to all DNA recombinants that include all or part of either the btPGSI208 gene or the btPGSI245 gene and that are suitable for transforming microorganisms (e.g., plant associated bacteria such as Bacillus subtilis, Pseudomonas, and Xanthomonas or yeasts such as Streptomyces cerevisiae) under conditions which enable all or part of the gene to be expressed and to be recoverable from said microorganisms or to be transferred to a plant cell.

References

- Botterman and Zabeau, DNA 6, 583-591 (1987)
- Deblaere, R., Bijtebier, B. De Greve , H., Debock, F., Schell, J., Van Montagu, M. and Leemans, J., Nucleic Acids Research 13, 4777-4788 (1985).
- Dulmage, H.T., "Production of Bacteria for Biological Control of Insects" in Biological Control in Crop Production, Ed. Paparizas, D.C., Osmun Publishers, Totowa, N.J., USA, pp. 129-141 (1981).
- Ellar, D.J., Knowles, B.H., Drobniewski, S.A. and Haider, M.Z., in "Fundamental and Applied aspects of Invertebrate Pathology". Ed. Samson, R.A., Vlak, J.M. and Peters, D. (1986) pp. 7-10. Wageningen, Foundation of the fourth International Colloqium of Invertebrate Pathology.
- Engvall and Pesce, Scand. Immunol. Suppl. 7 (1978)
- Finney, Probit Analysis, 3rd Edition, Cambridge University Press (1971)

- Franck, Guilley, Jonard, Richards and Hirth, Cell 21, 285-294 (1980)
- French, B.T., Maul, H.N. and Maul, G.G., Anal.Biochem. 156, 417423 (1986)
- Gardner, Howarth, Hahn, Brown-Luedi, Shepard and Messing, Nucleic Acids Research 9, 2871-2887 (1981)
- Gielen, J., De Beukeleer, M., Seurinck, J., Deboeck, F., De Greve, H., Lemmers, M., Van Montagu, M. and Schell, J., EMBO J 3, 835-845 (1984).
- Goldberg, R.B., Science 240, 1460-1467 (1988)
- Höfte, H., De Greve, H., Seurinck, J., Jansens, S., Mahillon, J., Ampe, Vandekerckhove, J, Vanderbruggen, H., Van Montagu, M., Zabeau, M. and Vaeck, M., Eur. J. Biochem. 161, 273-280 (1986)
- Höfte, H., Seurinck, J., Van Houtven A. and Vaeck, M., Nucleic Acids Research 15, 7183 (1987)
- Höfte, H., Dissertation thesis at the State University of Ghent, Belgium (1988).
- Höfte, H., Van Rie, J., Jansens, S., Van Houtven, A., Verbruggen, H. and Vaeck, M., Applied and Environmental Microbiology 54, 2010-2017 (1988)
- Hull and Howell, Virology 86, 482-493 (1987)
- Kuhlemeier, Green and Chua, Ann. Rev. Plant Physiol. 38, 221-257 (1987)
- Laemmli V., Nature 227, 680-685 (1970)
- Lambert, B., Leyns, F., Van Rooyen, L., Gosselé, F., Papon, Y. and Swings, J. Applied and Environmental Microbiology 53, 1866-1871 (1987).
- Mahillon, J. and Delcour, J., J. Microbiol. Methods 3, 69-73 (1984)
- Maxam, A.M. and Gilbert, W., Methods in Enzymol. 65, 499-560 (1980).
- Odell, J.T., Nagy, J., and Chua, N., Nature 313, 810-812 (1985).
- Peferoen, M. in Methods in Molecular Biology, vol. 3 : New Protein Techniques, p. 395-402, Ed. John M. Walker, Humana Press (1988).
- Reiss, B., Sprengel, R., Will, H. and Schaller, H., Gene 30, 217-223 (1984)
- Sneath, P., Mair, N., Sharpe, M. and Holt, J., (1986) in Bergey's Manual of Systematic Bacteriologie, Vol. 2, pp. 1104-1139. Eds. Williams and Wilkins, Baltimore, London.
- Sutherland, M.W. and Skerritt, J.M., Electrophoresis, 7, 401-406 (1986)
- Vaeck, M., Reynaerts, A., Höfte, H., Jansens, S., De Beuckeleer, M., Dean, C., Zabeau, M., Van Montagu, M. and Leemans, J., Nature 327, 33-37(1987).
- Velten, J., Velten, L., Hain, R. and Schell, J., EMBO J 3, 2723-2730 (1984).
- Velten, J. and Schell, J. Nucleic Acids Research 13, 6981-6998 (1985)
- Yannisch-Perron, C., Vierra, J. and Messing, J., Gene 33, 103-119 (1985).

## Claims

1. The BtPGSI208 strain or the BtPGSI245 strain.

2. The BtPGSI208 or BtPGSI245 crystals, crystal proteins, protoxin or toxin.

3. The btPGSI208 or btPGSI245 gene, the insecticidally effective btPGSI208 or btPGSI245 gene part, the truncated btPGSI208 or btPGSI245 gene, the btPGSI208 or btPGSI245 chimaeric gene or a hybrid thereof with a selectable marker gene, such as the neo gene.

4. An insecticidal composition, particularly against Coleoptera, which comprises an active ingredient selected from the group consisting of: the BtPGSI208 or BtPGSI245 strain, crystals, crystal proteins, protoxin and toxin and insecticidally effective portions of the BtPGSI208 or BtPGSI245 protoxin.

5. A transformed microorganism, particularly E. coli, characterized by the gene, the gene part, the truncated gene, the chimaeric gene or the hybrid of claim 3.

6. A transformed plant cell, characterized by the insecticidally effective btPGSI208 or btPGSI245 gene part, the truncated btPGSI208 or btPGSI245 gene, the btPGSI208 or btPGSI245 chimaeric gene, or a hybrid thereof with a selectable marker gene, such as the neo gene.

7. A plant or a seed thereof containing the plant cell of claim 6.

8. A plant genome containing, integrated therein, the gene part, the truncated gene or the chimaeric gene of claim 6.

9. A plant tissue, the cells of which have the plant genome of claim 8.

10. A process for rendering a plant resistant to Coleoptera characterized by: providing the plant with the transformed cell of claim 6.

11. In a process for producing plants and reproduction material, such as seeds, of said plants including a heterologous genetic material stably integrated in the genome thereof and capable of being expressed therein in the form of a protein toxic to insects, comprising the non-biological steps of: a) producing transformed plants cells or plant tissue including said heterologous genetic material from starting plant cells or plant tissue not expressing said protein, b) producing regenerated plants or reproduction material of said plants or both from said transformed plant cells or plant tissue including said heterologous genetic material, and c) optionally, biologically replicating said regenerated plants or reproduction material or both; wherein said step of producing said transformed plant cells or plant tissue including said heterologous genetic material is characterized by transforming said starting plant cells or plant tissue with the gene part, the truncated gene or the hybrid of claim 6 as well as regulatory elements which are capable of enabling the expression of the gene part, the truncated gene or the hybrid in said plant cells or plant tissue, to cause the stable integration of the gene part, the truncated gene or the hybrid in transformed plant cells or plant tissue, as well as in said plants and reproduction material produced therefrom throughout generations.

12. A process for controlling an insect pest, especially, Coleoptera, particularly Leptinotarsa decemlineata, Agelastica alni, Diabrotica luteola, Haltica tombacina Anthonomus grandis, Tenebrio molitor and Triboleum castaneum, characterized by contacting the pest with the insecticidal composition of claim 4.

15

EP 0 382 990 A1

```
                10         20         30          40         50         60         70

TTATCTACAT TTAATCATCT CCATTATAAA TATAATATCT AAACACATTG TTGCAAGAGA ATTAATACTA


                80         90         100        110        120        130        140

CTTTGATATT TTTAATATAC TTAACCTAAT GTATTGTTAA GTTAATATAG AAATATACCT ATATTAACAA


                150        160        170        180        190        200        210

GGAATTTATT AAAAATAATT TTGTATACTT TTCATTGTAA TAACATGATT TTTAAAACAA AAAAGTGTAT


                220        230        240        250      ·  260        270        280

AAACAACTTA TCAGGAAGGG GGGGATGCGC AAAGAATAAA AAGAGAATGC TTATAATGTT CAATGGTTTT


                290        300        310        320        330        340
                                                                             >_
ATAGGAAGGC ATTTTATCAG GTAGAAAGTT ATGTATTATG ATAAGAATGG GAGGAAGAAA A ATG
                                                                       MET
```

```
        350        359        368        377        386        395

AAT CCA AAC AAT CGA AGT GAA TAT GAT ACG ATA AAG GTT ACA CCT AAC AGT GAA
Asn Pro Asn Asn Arg Ser Glu Tyr Asp Thr Ile Lys Val Thr Pro Asn Ser Glu


        404        413        422        431        440        449

TTG CCA ACT AAC CAT AAT CAA TAT CCT TTA GCT GAC AAT CCA AAT TCG ACA CTA
Leu Pro Thr Asn His Asn Gln Tyr Pro Leu Ala Asp Asn Pro Asn Ser Thr Leu


        458        467        476        485        494        503

GAA GAA TTA AAT TAT AAA GAA TTT TTA AGA ATG ACT GCA GAC AAT TCT ACG GAA
Glu Glu Leu Asn Tyr Lys Glu Phe Leu Arg MET Thr Ala Asp Asn Ser Thr Glu


        512        521        530        539        548        557
          ↓
GTG CTA GAC AGC TCT ACA GTA AAA GAT GCA GTT GGG ACA GGA ATT TCT GTT GTA
Val Leu Asp Ser Ser Thr Val Lys Asp Ala Val Gly Thr Gly Ile Ser Val Val


        566        575        584        593        602        611

GGA CAG ATT TTA GGT GTT GTA GGG GTT CCA TTT GCT GGG GCG CTC ACT TCA TTT
Gly Gln Ile Leu Gly Val Val Gly Val Pro Phe Ala Gly Ala Leu Thr Ser Phe


        620        629        638        647        656        665

TAT CAA TCA TTT CTT AAC GCT ATA TGG CCA AGT GAT GCT GAC CCA TGG AAG GCT
Tyr Gln Ser Phe Leu Asn Ala Ile Trp Pro Ser Asp Ala Asp Pro Trp Lys Ala


        674        683        692        701        710        719

TTT ATG GCA CAA GTG GAA GTA CTG ATA GAT AAG AAA ATA GAG GAG TAT GCT AAA
Phe MET Ala Gln Val Glu Val Leu Ile Asp Lys Lys Ile Glu Glu Tyr Ala Lys
```

FIGURE 1

```
     728         737         746         755         764         773
    --- --- --- --- --- --- --- --- --- --- --- --- --- --- --- ---
    AGT AAA GCT CTT GCA GAG TTA CAG GGT CTT CAA AAT AAT TTT GAA GAT TAT GTA
    Ser Lys Ala Leu Ala Glu Leu Gln Gly Leu Gln Asn Asn Phe Glu Asp Tyr Val


     782         791         800         809         818         827
    --- --- --- --- --- --- --- --- --- --- --- --- --- --- --- ---
    AAT GCG TTG GAT TCC TGG AAG AAA GCG CCT GTA AAT TTA CGA AGT CGA AGA AGC
    Asn Ala Leu Asp Ser Trp Lys Lys Ala Pro Val Asn Leu Arg Ser Arg Arg Ser


     836         845         854         863         872         881
    --- --- --- --- --- --- --- --- --- --- --- --- --- --- --- ---
    CAA GAT CGA ATA AGA GAA CTT TTT TCT CAA GCA GAA AGC CAT TTT CGT AAT TCC
    Gln Asp Arg Ile Arg Glu Leu Phe Ser Gln Ala Glu Ser His Phe Arg Asn Ser


     890         899         908         917      ·  926         935
    --- --- --- --- --- --- --- --- --- --- --- --- --- --- --- ---
    ATG CCG TCA TTT GCG GTT TCC AAA TTC GAA GTT CTG TTT CTA CCA ACA TAT GCA
    MET Pro Ser Phe Ala Val Ser Lys Phe Glu Val Leu Phe Leu Pro Thr Tyr Ala


     944         953         962         971         980         989
    --- --- --- --- --- --- --- --- --- --- --- --- --- --- --- ---
    CAA GCT GCA AAT ACA CAT TTA TTG CTA TTA AAA GAT GCT CAA GTT TTT GGA GAA
    Gln Ala Ala Asn Thr His Leu Leu Leu Leu Lys Asp Ala Gln Val Phe Gly Glu


     998        1007        1016        1025        1034        1043
    --- --- --- --- --- --- --- --- --- --- --- --- --- --- --- ---
    GAA TGG GGA TAT TCT TCA GAA GAT ATT GCT GAA TTT TAT CAA AGA CAA TTA AAA
    Glu Trp Gly Tyr Ser Ser Glu Asp Ile Ala Glu Phe Tyr Gln Arg Gln Leu Lys


    1052        1061        1070        1079        1088        1097
    --- --- --- --- --- --- --- --- --- --- --- --- --- --- --- ---
    CTT ACG CAA CAA TAC ACT GAC CAT TGT GTC AAT TGG TAT AAT GTT GGA TTA AAT
    Leu Thr Gln Gln Tyr Thr Asp His Cys Val Asn Trp Tyr Asn Val Gly Leu Asn


    1106        1115        1124        1133        1142        1151
    --- --- --- --- --- --- --- --- --- --- --- --- --- --- --- ---
    AGT TTA AGA GGT TCA ACT TAT GAT GCA TGG GTC AAA TTT AAC CGT TTT CGC AGA
    Ser Leu Arg Gly Ser Thr Tyr Asp Ala Trp Val Lys Phe Asn Arg Phe Arg Arg


    1160        1169        1178        1187        1196        1205
    --- --- --- --- --- --- --- --- --- --- --- --- --- --- --- ---
    GAA ATG ACA TTA ACT GTA TTA GAT CTA ATT GTA TTA TTC CCA TTT TAT GAT GTT
    Glu MET Thr Leu Thr Val Leu Asp Leu Ile Val Leu Phe Pro Phe Tyr Asp Val


    1214        1223        1232        1241        1250        1259
    --- --- --- --- --- --- --- --- --- --- --- --- --- --- --- ---
    CGG TTA TAC TCA AAA GGA GTT AAA ACA GAA CTA ACA AGA GAC ATT TTT ACA GAT
    Arg Leu Tyr Ser Lys Gly Val Lys Thr Glu Leu Thr Arg Asp Ile Phe Thr Asp


    1268        1277        1286        1295        1304        1313
    --- --- --- --- --- --- --- --- --- --- --- --- --- --- --- ---
    CCA ATT TTT ACA CTC AAT GCT CTT CAA GAG TAT GGA CCA ACT TTT TCG AGT ATA
    Pro Ile Phe Thr Leu Asn Ala Leu Gln Glu Tyr Gly Pro Thr Phe Ser Ser Ile


    1322        1331        1340        1349        1358        1367
    --- --- --- --- --- --- --- --- --- --- --- --- --- --- --- ---
    GAA AAC TCT ATT CGA AAA CCT CAT TTA TTT GAT TAT TTG CGT GGG ATT GAA TTT
    Glu Asn Ser Ile Arg Lys Pro His Leu Phe Asp Tyr Leu Arg Gly Ile Glu Phe
```

Figure 1 (cont. 1)

EP 0 382 990 A1

```
        1376        1385        1394        1403        1412        1421

CAT ACG CGT CTT CGA CCT GGT TAC TCT GGG AAA GAT TCT TTC AAT TAT TGG TCT
His Thr Arg Leu Arg Pro Gly Tyr Ser Gly Lys Asp Ser Phe Asn Tyr Trp Ser

        1430        1439        1448        1457        1466        1475

GGT AAT TAT GTA GAA ACT AGA CCT AGT ATA GGA TCT AAT GAT ACA ATC ACT TCC
Gly Asn Tyr Val Glu Thr Arg Pro Ser Ile Gly Ser Asn Asp Thr Ile Thr Ser

        1484        1493        1502        1511        1520        1529

CCA TTT TAT GGA GAT AAA TCT ATT GAA CCT ATA CAA AAG CTA AGC TTT GAT GGA
Pro Phe Tyr Gly Asp Lys Ser Ile Glu Pro Ile Gln Lys Leu Ser Phe Asp Gly

        1538        1547        1556        1565   ·    1574        1583

CAA AAA GTT TAT CGA ACT ATA GCT AAT ACA GAC ATA GCG GCT TTT CCG GAT GGC
Gln Lys Val Tyr Arg Thr Ile Ala Asn Thr Asp Ile Ala Ala Phe Pro Asp Gly

        1592        1601        1610        1619        1628        1637

AAG ATA TAT TTT GGT GTT ACG AAA GTT GAT TTT AGT CAA TAT GAT GAT CAA AAA
Lys Ile Tyr Phe Gly Val Thr Lys Val Asp Phe Ser Gln Tyr Asp Asp Gln Lys

        1646        1655        1664        1673        1682        1691

AAT GAA ACT AGT ACA CAA ACA TAT GAT TCA AAA AGA TAC AAT GGC TAT TTA GGT
Asn Glu Thr Ser Thr Gln Thr Tyr Asp Ser Lys Arg Tyr Asn Gly Tyr Leu Gly

        1700        1709        1718        1727        1736        1745

GCA CAG GAT TCT ATC GAC CAA TTA CCA CCA GAA ACA ACA GAT GAA CCA CTT GAA
Ala Gln Asp Ser Ile Asp Gln Leu Pro Pro Glu Thr Thr Asp Glu Pro Leu Glu

        1754        1763        1772        1781        1790        1799

AAA GCA TAT AGT CAT CAG CTT AAT TAC GCA GAA TGT TTC TTA ATG CAG GAC CGT
Lys Ala Tyr Ser His Gln Leu Asn Tyr Ala Glu Cys Phe Leu MET Gln Asp Arg

        1808        1817        1826        1835        1844        1853

CGT GGA ACA ATT CCA TTT TTT ACT TGG ACA CAT AGA AGT GTA GAC TTT TTT AAT
Arg Gly Thr Ile Pro Phe Phe Thr Trp Thr His Arg Ser Val Asp Phe Phe Asn

        1862        1871        1880        1889        1898        1907

ACA ATT GAT GCT GAA AAA ATT ACT CAA CTT CCA GTA GTG AAA GCA TAT GCC TTG
Thr Ile Asp Ala Glu Lys Ile Thr Gln Leu Pro Val Val Lys Ala Tyr Ala Leu

        1916        1925        1934        1943        1952        1961

TCT TCA GGC GCT TCC ATT ATT GAA GGT CCA GGA TTC ACA GGA GGA AAT TTA CTA
Ser Ser Gly Ala Ser Ile Ile Glu Gly Pro Gly Phe Thr Gly Gly Asn Leu Leu

        1970        1979        1988        1997        2006        2015

TTC CTA AAA GAA TCT AGT AAT TCA ATT GCT AAA TTT AAA GTT ACC TTA AAT TCA
Phe Leu Lys Glu Ser Ser Asn Ser Ile Ala Lys Phe Lys Val Thr Leu Asn Ser
```

Figure 1 (cont. 2)

2024        2033        2042        2051        2060        2069

GCA GCC TTG TTA CAA CGA TAT CGC GTA AGA ATA CGC TAT GCT TCA ACC ACT AAC
Ala Ala Leu Leu Gln Arg Tyr Arg Val Arg Ile Arg Tyr Ala Ser Thr Thr Asn

2078        2087        2096        2105        2114        2123

CTA CGA CTT TTC GTG CAA AAT TCA AAC AAT GAT TTT CTT GTC ATC TAC ATT AAT
Leu Arg Leu Phe Val Gln Asn Ser Asn Asn Asp Phe Leu Val Ile Tyr Ile Asn

2132        2141        2150        2159        2168        2177

AAA ACT ATG AAT ATA GAT GGT GAT TTA ACA TAT CAA ACA TTT GAT TTC GCA ACT
Lys Thr MET Asn Ile Asp Gly Asp Leu Thr Tyr Gln Thr Phe Asp Phe Ala Thr

2186        2195        2204        2213        2222        2231

AGT AAT TCT AAT ATG GGA TTC TCT GGT GAT ACA AAT GAC TTT ATA ATA GGA GCA
Ser Asn Ser Asn MET Gly Phe Ser Gly Asp Thr Asn Asp Phe Ile Ile Gly Ala

2240        2249        2258        2267        2276        2285

GAA TCT TTC GTT TCT AAT GAA AAA ATC TAT ATA GAT AAG ATA GAA TTT ATC CCA
Glu Ser Phe Val Ser Asn Glu Lys Ile Tyr Ile Asp Lys Ile Glu Phe Ile Pro

2294            2307    2317    2327    2337    2347    2357

GTA CAA TAG CAAGTGAATT TTGGAATATA GGGCGATGGT CAAAATGAAA GGATAAGAAG GTGAATTTTG
Val Gln  .

2367    2377    2387    2397    2407    2417

ATGGTTAGGA AAGATTCTTT TAACAAAAGC AACATGGAAA AGTATACAGT ACAAATGGGT ACCGAGCT


Figure 1 (cont. 3)

Figure 2.

TTTGGATTGT GAGCATGTAC AGGTTTGTGA TTTACAAGCA AAACCAATCT GCGAAGATTG TTGTCATTTT

ATAAAGGTAA CAGGATATTT TCAAATTTGT ACCGATTAAA TAAAAAATAT TTAGATTAAC ACTGTTGTTT

TTTACAACTA TCCGTATGGA CAAATTTAAC AAGGAGTGAA AAT ATG AAT TTA AAT AAT TTA
                                              MET Asn Leu Asn Asn Leu

GAT GGA TAT GAA GAT AGT AAT AGA ACA TTA AAT AAT TCT CTC AAT TAT CCT ACT
Asp Gly Tyr Glu Asp Ser Asn Arg Thr Leu Asn Asn Ser Leu Asn Tyr Pro Thr

CAA AAA GCA TTA TCA CCA TCA TTA AAG AAT ATG AAC TAC CAG GAT TTT TTA TCT
Gln Lys Ala Leu Ser Pro Ser Leu Lys Asn MET Asn Tyr Gln Asp Phe Leu Ser

ATA ACT GAG AGG GAA CAA CCT GAA GCA CTC GCT AGT GGT AAT ACA GCT ATT AAT
Ile Thr Glu Arg Glu Gln Pro Glu Ala Leu Ala Ser Gly Asn Thr Ala Ile Asn

ACT GTA GTT AGT GTT ACG GGG GCT ACA CTA AGT GCG TTA GGT GTC CCA GGT GCA
Thr Val Val Ser Val Thr Gly Ala Thr Leu Ser Ala Leu Gly Val Pro Gly Ala

AGT TTT ATC ACT AAC TTT TAC CTG AAA ATT GCA GGC CTT TTA TGG CCA GAA AAT
Ser Phe Ile Thr Asn Phe Tyr Leu Lys Ile Ala Gly Leu Leu Trp Pro Glu Asn

GGA AAA ATT TGG GAT GAA TTT ATG ACA GAA GTA GAA GCA CTT ATT GAT CAA AAA
Gly Lys Ile Trp Asp Glu Phe MET Thr Glu Val Glu Ala Leu Ile Asp Gln Lys

ATA GAA GAA TAT GTA AGA AAT AAA GCG ATT GCA GAA TTA GAT GGA TTA GGA TCA
Ile Glu Glu Tyr Val Arg Asn Lys Ala Ile Ala Glu Leu Asp Gly Leu Gly Ser

GCC TTA GAT AAA TAT CAA AAA GCA CTT GCA GAT TGG CTG GGC AAA CAA GAT GAT
Ala Leu Asp Lys Tyr Gln Lys Ala Leu Ala Asp Trp Leu Gly Lys Gln Asp Asp

CCA GAA GCT ATA CTT TCT GTG GCA ACT GAA TTT CGT ATA ATA GAT TCT CTT TTT
Pro Glu Ala Ile Leu Ser Val Ala Thr Glu Phe Arg Ile Ile Asp Ser Leu Phe

Figure 2 (cont. 1)

```
        693       702       711       720       729       738

GAA TTT AGT ATG CCT TCA TTT AAG GTT ACT GGA TAT GAA ATA CCA TTA CTA ACA
Glu Phe Ser MET Pro Ser Phe Lys Val Thr Gly Tyr Glu Ile Pro Leu Leu Thr


        747       756       765       774       783       792

GTT TAC GCA CAA GCG GCA AAC CTT CAT CTA GCT TTA TTA AGA GAT TCT ACT CTT
Val Tyr Ala Gln Ala Ala Asn Leu His Leu Ala Leu Leu Arg Asp Ser Thr Leu


        801       810       819       828       837       846

TAT GGA GAT AAA TGG GGA TTC ACT CAG AAC AAC ATT GAG GAA AAT TAT AAT CGT
Tyr Gly Asp Lys Trp Gly Phe Thr Gln Asn Asn Ile Glu Glu Asn Tyr Asn Arg


        855       864       873       882       891       900

CAA AAG AAA CGC ATT TCT GAA TAT TCA GAC CAT TGC ACC AAG TGG TAT AAT AGT
Gln Lys Lys Arg Ile Ser Glu Tyr Ser Asp His Cys Thr Lys Trp Tyr Asn Ser


        909       918       927       936       945       954

GGT CTT AGC AGA TTG AAC GGT TCC ACT TAT GAA CAA TGG ATA AAT TAT AAT CGT
Gly Leu Ser Arg Leu Asn Gly Ser Thr Tyr Glu Gln Trp Ile Asn Tyr Asn Arg


        963       972       981       990       999      1008

TTT CGT AGA GAA ATG ATA TTA ATG GCA TTA GAT CTT GTC GCT GTA TTT CCT TTT
Phe Arg Arg Glu MET Ile Leu MET Ala Leu Asp Leu Val Ala Val Phe Pro Phe


       1017      1026      1035      1044      1053      1062

CAT GAC CCT CGA AGG TAT TCA ATG GAA ACA AGT ACG CAG TTA ACG AGA GAA GTG
His Asp Pro Arg Arg Tyr Ser MET Glu Thr Ser Thr Gln Leu Thr Arg Glu Val


       1071      1080      1089      1098      1107      1116

TAT ACC GAT CCA GTT AGC TTG TCA ATT AGC AAT CCA GAT ATA GGT CCA AGT TTT
Tyr Thr Asp Pro Val Ser Leu Ser Ile Ser Asn Pro Asp Ile Gly Pro Ser Phe


       1125      1134      1143      1152      1161      1170

TCT CAG ATG GAA AAT ACT GCA ATT AGA ACA CCA CAC CTT GTT GAT TAT TTA GAT
Ser Gln MET Glu Asn Thr Ala Ile Arg Thr Pro His Leu Val Asp Tyr Leu Asp


       1179      1188      1197      1206      1215      1224

GAG CTT TAT ATA TAT ACA TCA AAA TAT AAA GCA TTT TCA CAT GAG ATT CAA CCA
Glu Leu Tyr Ile Tyr Thr Ser Lys Tyr Lys Ala Phe Ser His Glu Ile Gln Pro


       1233      1242      1251      1260      1269      1278

GAC CTA TTT TAT TGG AGT GCA CAT AAG GTT AGC TTT AAA AAA TCG GAG CAA TCC
Asp Leu Phe Tyr Trp Ser Ala His Lys Val Ser Phe Lys Lys Ser Glu Gln Ser


       1287      1296      1305      1314      1323      1332

AAT TTA TAT ACA ACA GGC ATA TAT GGT AAA ACA AGT GGA TAT ATT TCA TCA GGG
Asn Leu Tyr Thr Thr Gly Ile Tyr Gly Lys Thr Ser Gly Tyr Ile Ser Ser Gly
```

Figure 2
(cont. 2)

```
GCA TAT TCA TTT CAT GGG AAT GAT ATC TAT AGA ACA TTA GCA GCT CCA TCA GTT
Ala Tyr Ser Phe His Gly Asn Asp Ile Tyr Arg Thr Leu Ala Ala Pro Ser Val

GTA GTT TAT CCG TAT ACT CAG AAT TAT GGT GTC GAG CAA GTT GAG TTT TAC GGT
Val Val Tyr Pro Tyr Thr Gln Asn Tyr Gly Val Glu Gln Val Glu Phe Tyr Gly

GTA AAA GGG CAT GTA CAT TAT AGA GGA GAT AAC AAA TAT GAT CTG ACG TAT GAT
Val Lys Gly His Val His Tyr Arg Gly Asp Asn Lys Tyr Asp Leu Thr Tyr Asp

TCT ATT GAT CAA TTA CCC CCA GAC GGA GAA CCA ATA CAC GAA AAA TAC ACT CAT
Ser Ile Asp Gln Leu Pro Pro Asp Gly Glu Pro Ile His Glu Lys Tyr Thr His

CGA TTA TGT CAT GCT ACA GCT ATA TTT AAA TCA ACT CCG GAT TAT GAT AAT GCT
Arg Leu Cys His Ala Thr Ala Ile Phe Lys Ser Thr Pro Asp Tyr Asp Asn Ala

ACT ATC CCG ATC TTT TCT TGG ACG CAT AGA AGT GCG GAG TAT TAC AAT AGA ATC
Thr Ile Pro Ile Phe Ser Trp Thr His Arg Ser Ala Glu Tyr Tyr Asn Arg Ile

TAT CCA AAC AAA ATC ACA AAA ATT CCA GCT GTA AAA ATG TAT AAA CTA GAT GAT
Tyr Pro Asn Lys Ile Thr Lys Ile Pro Ala Val Lys MET Tyr Lys Leu Asp Asp

CCA TCT ACA GTT GTC AAA GGG CCT GGA TTT ACA GGT GGA GAT TTA GTT AAG AGA
Pro Ser Thr Val Val Lys Gly Pro Gly Phe Thr Gly Gly Asp Leu Val Lys Arg

GGG AGT ACT GGT TAT ATA GGA GAT ATA AAG GCT ACC GTA AAC TCT CCA CTT TCT
Gly Ser Thr Gly Tyr Ile Gly Asp Ile Lys Ala Thr Val Asn Ser Pro Leu Ser

CAA AAA TAT CGT GTT AGA GTT CGA TAC GCT ACT AAT GTT TCT GGA CAA TTC AAC
Gln Lys Tyr Arg Val Arg Val Arg Tyr Ala Thr Asn Val Ser Gly Gln Phe Asn

GTG TAT ATT AAT GAT AAA ATA ACG CTT CAA ACA AAG TTT CAA AAT ACT GTA GAA
Val Tyr Ile Asn Asp Lys Ile Thr Leu Gln Thr Lys Phe Gln Asn Thr Val Glu

ACA ATA GGT GAA GGA AAA GAT TTA ACC TAT GGT TCA TTT GGA TAT ATA GAA TAT
Thr Ile Gly Glu Gly Lys Asp Leu Thr Tyr Gly Ser Phe Gly Tyr Ile Glu Tyr

TCT ACG ACC ATT CAA TTT CCG GAT GAG CAT CCA AAA ATC ACT CTT CAT TTA AGC
Ser Thr Thr Ile Gln Phe Pro Asp Glu His Pro Lys Ile Thr Leu His Leu Ser
```

Figure 2
(cont. 3)

2043    2052    2061    2070    2079    2088

```
GAT TTG AGT AAC AAT TCA TCA TTT TAT GTA GAT TCA ATC GAA TTT ATC CCT GTA
Asp Leu Ser Asn Asn Ser Ser Phe Tyr Val Asp Ser Ile Glu Phe Ile Pro Val
```

2097    2106    2115    2124    2133    2142

```
GAT GTA AAT TAT GCT GAA AAA GAA AAA CTA GAA AAA GCA CAG AAA GCC GTG AAT
Asp Val Asn Tyr Ala Glu Lys Glu Lys Leu Glu Lys Ala Gln Lys Ala Val Asn
```

2151    2160    2169    2178    2187    2196

```
ACC TTG TTT ACA GAG GGA AGA AAT GCA CTC CAA AAA GAC GTG ACA GAT TAT AAA
Thr Leu Phe Thr Glu Gly Arg Asn Ala Leu Gln Lys Asp Val Thr Asp Tyr Lys
```

2205    2214    2223    2232    2241    2250

```
GTG GAC CAG GTT TCA ATT TTA GTG GAT TGT ATA TCA GGG GAT TTA TAT CCC AAT
Val Asp Gln Val Ser Ile Leu Val Asp Cys Ile Ser Gly Asp Leu Tyr Pro Asn
```

2259    2268    2277    2286    2295    2304

```
GAG AAA CGC GAA CTA CAA AAT CTA GTC AAA TAC GCA AAA CGT TTG AGC TAT TCC
Glu Lys Arg Glu Leu Gln Asn Leu Val Lys Tyr Ala Lys Arg Leu Ser Tyr Ser
```

2313    2322    2331    2340    2349    2358

```
CGT AAT TTA CTT CTA GAT CCA ACA TTC GAT TCT ATT AAT TCA TCT GAG GAG AAT
Arg Asn Leu Leu Leu Asp Pro Thr Phe Asp Ser Ile Asn Ser Ser Glu Glu Asn
```

2367    2376    2385    2394    2403    2412

```
GGT TGG TAT GGA AGT AAT GGT ATT GTG ATT GGA AAT GGG GAT TTT GTA TTC AAA
Gly Trp Tyr Gly Ser Asn Gly Ile Val Ile Gly Asn Gly Asp Phe Val Phe Lys
```

2421    2430    2439    2448    2457    2466

```
GGT AAC TAT TTA ATT TTT TCA GGT ACC AAT GAT ACA CAA TAT CCA ACA TAT CTC
Gly Asn Tyr Leu Ile Phe Ser Gly Thr Asn Asp Thr Gln Tyr Pro Thr Tyr Leu
```

2475    2484    2493    2502    2511    2520

```
TAC CAA AAA ATA GAT GAA TCC AAA CTC AAA GAA TAT ACA CGC TAT AAA CTG AAA
Tyr Gln Lys Ile Asp Glu Ser Lys Leu Lys Glu Tyr Thr Arg Tyr Lys Leu Lys
```

2529    2538    2547    2556    2565    2574

```
GGT TTT ATC GAA AGT AGT CAG GAT TTA GAA GCT TAT GTG ATT CGC TAT GAT GCA
Gly Phe Ile Glu Ser Ser Gln Asp Leu Glu Ala Tyr Val Ile Arg Tyr Asp Ala
```

2583    2592    2601    2610    2619    2628

```
AAA CAT AGA ACA TTG GAT GTT TCT GAT AAT CTA TTA CCA GAT ATT CTC CCT GAG
Lys His Arg Thr Leu Asp Val Ser Asp Asn Leu Leu Pro Asp Ile Leu Pro Glu
```

2637    2646    2655    2664    2673    2682

```
AAT ACA TGT GGA GAA CCA AAT CGC TGC GCG GCA CAA CAA TAC CTG GAT GAA AAT
Asn Thr Cys Gly Glu Pro Asn Arg Cys Ala Ala Gln Gln Tyr Leu Asp Glu Asn
```

2691    2700    2709    2718    2727    2736

```
CCA AGT CCA GAA TGT AGT TCG ATG CAA GAT GGA ATT TTG TCT GAT TCG CAT TCA
Pro Ser Pro Glu Cys Ser Ser MET Gln Asp Gly Ile Leu Ser Asp Ser His Ser
```

Figure 2
(cont.4)

TTT TCT CTT AAT ATA GAT ACA GGT TCT ATC AAT CAC AAT GAG AAT TTA GGA ATT
Phe Ser Leu Asn Ile Asp Thr Gly Ser Ile Asn His Asn Glu Asn Leu Gly Ile

TGG GTG TTG TTT AAA ATT TCG ACA TTA GAA GGA TAT GCG AAA TTT GGA AAT CTA
Trp Val Leu Phe Lys Ile Ser Thr Leu Glu Gly Tyr Ala Lys Phe Gly Asn Leu

GAA GTG ATT GAA GAT GGC CCA GTT ATT GGA GAA GCA TTA GCC CGT GTG AAA CGC
Glu Val Ile Glu Asp Gly Pro Val Ile Gly Glu Ala Leu Ala Arg Val Lys Arg

CAA GAA ACG AAG TGG AGA AAC AAG TTA GCC CAA CTG ACA ACG GAA ACA CAA GCG
Gln Glu Thr Lys Trp Arg Asn Lys Leu Ala Gln Leu Thr Thr Glu Thr Gln Ala

ATT TAT ACA CGA GCA AAA CAA GCG CTG GAT AAT CTT TTT GCG AAT GCA CAA GAC
Ile Tyr Thr Arg Ala Lys Gln Ala Leu Asp Asn Leu Phe Ala Asn Ala Gln Asp

TCT CAC TTA AAA AGA GAT GTT ACA TTT GCG GAA ATT GCG GCT GCA AGA AAG ATT
Ser His Leu Lys Arg Asp Val Thr Phe Ala Glu Ile Ala Ala Ala Arg Lys Ile

GTC CAA TCA ATA CGC GAA GCG TAT ATG TCA TGG TTA TCT GTT GTT CCA GGT GTA
Val Gln Ser Ile Arg Glu Ala Tyr MET Ser Trp Leu Ser Val Val Pro Gly Val

AAT CAC CCT ATT TTT ACA GAG TTA AGT GGG CGA GTA CAA CGA GCA TTT CAA TTA
Asn His Pro Ile Phe Thr Glu Leu Ser Gly Arg Val Gln Arg Ala Phe Gln Leu

TAT GAT GTA CGA AAT GTT GTG CGT AAT GGT CGA TTC CTC AAT GGC TTA TCC GAT
Tyr Asp Val Arg Asn Val Val Arg Asn Gly Arg Phe Leu Asn Gly Leu Ser Asp

TGG ATT GTA ACA TCT GAC GTA AAG GTA CAA GAA GAA AAT GGG AAT AAC GTA TTA
Trp Ile Val Thr Ser Asp Val Lys Val Gln Glu Glu Asn Gly Asn Asn Val Leu

GTT CTT AAC AAT TGG GAT GCA CAA GTA TTA CAA AAC GTA AAA CTC TAT CAA GAC
Val Leu Asn Asn Trp Asp Ala Gln Val Leu Gln Asn Val Lys Leu Tyr Gln Asp

CGT GGG TAT ATC TTA CAT GTA ACA GCG CGC AAG ATA GGA ATT GGG GAA GGA TAT
Arg Gly Tyr Ile Leu His Val Thr Ala Arg Lys Ile Gly Ile Gly Glu Gly Tyr

Figure 2
(cont.5)

```
      3393        3402        3411        3420          3429        3438

ATA ACG ATT ACG GAT GAA GAA GGG CAT ACA GAT CAA TTG AGA TTT ACT GCA TGT
Ile Thr Ile Thr Asp Glu Glu Gly His Thr Asp Gln Leu Arg Phe Thr Ala Cys


      3447        3456        3465        3474          3483        3492

GAA GAG ATT GAT GCA TCT AAT GCG TTT ATA TCC GGT TAT ATT ACA AAA GAA CTG
Glu Glu Ile Asp Ala Ser Asn Ala Phe Ile Ser Gly Tyr Ile Thr Lys Glu Leu


      3501        3510        3519        3528          3537        3546

GAA TTC TTC CCA GAT ACA GAG AAA GTG CAT ATA GAA ATA GGC GAA ACA GAA GGA
Glu Phe Phe Pro Asp Thr Glu Lys Val His Ile Glu Ile Gly Glu Thr Glu Gly


      3555        3564        3573        3582   *     3591        3600        3610
                                                                              >
ATA TTC CTG GTA GAA AGT ATA GAG TTA TTT TTG ATG GAA GAG CTA TGT TAA TAGGGAGATT
Ile Phe Leu Val Glu Ser Ile Glu Leu Phe Leu MET Glu Glu Leu Cys  .


        3620        3630        3640        3650        3660        3670        3680

ATTCAACAAA TATTTGTTTG ATTCAAAATA AAATAAAATG CATACAATCC TCTTTATCAG ACGGTATTTC


        3690        3700        3710        3720        3730        3740        3750

TAATAATTAT AAATATAGGT TGAAAGTTAA AAAATAAAAA CACGCTATTC CCATTACTAG AAGGAGGGAG


        3760        3770        3780        3790        3800        3810        3820

TAACGTGTTT TTTCATGAGT AAAAAAACAA TTAGCTATAT TTATCTATTC TCTATAGAAG AAGCGGATTG


        3830        3840        3850        3860        3870        3880        3890

ATAAGAACCG TAAGTGACAG GAATAGCATT TATATCTTAT AGTGCAAGTC CAAACAAATG AGGGTAGTAG


        3900        3910        3920        3930        3940        3950        3960

AGTGACAAAA ACGCTTGAAG TTTTCCAAAA AAGAAATCAA GTACAAATTG AAATTAGTAC AACAAATGTT


        3970        3980        3990        4000

ATTTCTTTAG TAGAACGTAT AGAATTATTA TGTTTGGAAG ATGA
```

Total protein pattern of
sporulated B.t.s by SDS-PAGE

Figure 3

Protein blotting of BtS1 and PGSI208
proteins and trypsinized crystal proteins

PROTEIN PATTERN          ANTI-Cry III A

# Figure 4a

Protein blotting of HD110, PGSI245 and BtS1
solubilized and trypsinized crystal proteins

PROTEIN PATTERN    ANTI-Cry IIIA    ANTI-Cry IA(b)

# Figure 4b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 808 880 (ECOGEN INCORPORATED) * abstract; page 13, line 25 - page 14, line 29; page 38, line 30 - page 39, line 35; page 41, line 30 - page 42, line 30; figure 8; claims 1-5,8-14,17-25,28-54,60,62,65-67 * | 1-12 | C 12 N 15/32 A 01 N 63/00 |
| D,A | EP-A-0 213 818 (MYCOGEN CORPORATION) * abstract; column 2, lines 5-17; column 3, line 54 - column 4, line 54; column 5, lines 1-37; column 6, lines 1-16; column 6, lines 31-34; examples 2-5; tables A,B; claims * | 2-5,12 | |
| A | EP-A-0 289 479 (MONSANTO COMPANY) * whole document * | 1-12 | |
| A | PROCEEDINGS OF THE NATIONAL ACADEMY OF SCIENCES OF THE USA vol. 84, no. 20, October 1987, pages 7036-7040, Washington, DC, USA; V. SEKAR et al.: "Molecular cloning and characterization of the insecticidal crystal protein gene of Bacillus thuringiensis var. tenebrionis" * whole document * | 2-5,12 | TECHNICAL FIELDS SEARCHED (Int. Cl.5)  C 12 N 15/32 C 12 N 15/82 A 01 N 63/00 A 01 N 63/02 |
| A | BIOSIS DATABASE abstract no. 87-046633, 1987; W.P. DONOVAN et al.: "Isolation and characterization of EG2158 a new strain of Bacillus-Thuringiensis toxic to coleopteran larvae and nucleotide sequence of the toxin gene" & Molecular & General Genetics 1988, vol. 214, no. 3, pages 365-372 | 1-5 | |

-/-

The present search report has been drawn up for all claims

| Place of search | Date of completion of the search | Examiner |
|---|---|---|
| BERLIN | 27-09-1989 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 193 259 (PLANT GENETIC SYSTEMS N.V.)<br>* whole document * | 1-12 | |

TECHNICAL FIELDS SEARCHED (Int. Cl.5)

*The present search report has been drawn up for all claims*

| Place of search | Date of completion of the search | Examiner |
|---|---|---|
| BERLIN | 27-09-1989 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)